# EUROPEAN PATENT SPECIFICATION

(11) **EP 1 945 244 B1**
(45) Date of publication and mention of the grant of the patent: **04.05.2016**
(21) Application number: 06809758.3
(22) Date of filing: 16.10.2006
(51) Int. Cl.: A61K 38/48, A61K 38/18, A61K 38/55, A61K 31/7105

(54) **USE OF CASPASE-8 FOR PHARMACEUTICAL COMPOSITIONS AND DIAGNOSTIC METHODS FOR INFLAMMATORY SKIN DISESAES, DISORDERS OR CONDITIONS**
VERWENDUNG VON CASPASE-8 IN PHARMAZEUTISCHEN ZUSAMMENSETZUNGEN UND DIAGNOSEVERFAHREN FÜR ENTZÜNDLICHE HAUTERKRANKUNGEN, STÖRUNGEN ODER ZUSTÄNDE
UTILISATION DE LA CASPASE-8 DANS DES COMPOSITIONS PHARMACEUTIQUES ET DANS DES PROCÉDÉS DE DIAGNOSTIC DE MALADIES, AFFECTIONS OU ÉTATS CUTANÉS INFLAMMATOIRES

(30) Priority: 16.10.2005 IL 17145105
(43) Date of publication of application: 23.07.2008
(73) Proprietor: YEDA RESEARCH AND DEVELOPMENT CO., LTD., 76100 Rehovot (IL)
(72) Inventor: WALLACH, David, 76406 Rehovot (IL); KOVALENKO, Andrei, 76100 Rehovot (IL); KANG, Tae-Bong, Seoul, 121-882 (KR); KIM, Jin Chul, 76100 Rehovot (IL)
(74) Representative: Dennemeyer & Associates S.A.
(86) International application number: PCT/IL2006/001188
(87) International publication number: WO 2007/046087

(56) References cited:
- WO-A-00/51432
- WO-A-98/37901
- WO-A-2004/056868
- WO-A2-03/066661
- WO-A2-2005/001052
- WO-A2-2005/079844
- US-A1- 2001 006 793
- POSWIG A ET AL: "Short-term therapy with TNF alpha receptor improves psoriatic arthritis but not psoriasis integumentalis" H+G ZEITSCHRIFT FUR HAUTKRANKHEITEN 2000 GERMANY, vol. 75, no. 3, 2000, pages 153-154, XP008074236 ISSN: 0301-0481
- CHAUDHARY P M ET AL: "Activation of the NF-[kappa]B pathway by Caspase 8 and its homologs" ONCOGENE 14 SEP 2000 UNITED KINGDOM, vol. 19, no. 39, 14 September 2000 (2000-09-14), pages 4451-4460, XP002432875 ISSN: 0950-9232
- RIHS H P ET AL: "Rapid detection of the SPINK5 polymorphism Glu420Lys by real-time PCR technology" CLINICA CHIMICA ACTA, AMSTERDAM, NL, vol. 355, no. 1-2, May 2005 (2005-05), pages 185-189, XP004835642 ISSN: 0009-8981
- MASCIA F ET AL: "Blockade of the EGF receptor induces a deranged chemokine expression in keratinocytes leading to enhanced skin inflammation" AMERICAN JOURNAL OF PATHOLOGY, PHILADELPHIA, PA, US, vol. 163, no. 1, July 2003 (2003-07), pages 303-312, XP002410413 ISSN: 0002-9440
- FLIER J ET AL: "Differential expression of CXCR3 targeting chemokines CXCL10, CXCL9, and CXCL11 in different types of skin inflammation." THE JOURNAL OF PATHOLOGY AUG 2001, vol. 194, no. 4, August 2001 (2001-08), pages 398-405, XP002432876 ISSN: 0022-3417
- SEMPRINI SABRINA ET AL: "Evidence for differential S100 gene over-expression in psoriatic patients from genetically heterogeneous pedigrees." HUMAN GENETICS OCT 2002, vol. 111, no. 4-5, October 2002 (2002-10), pages 310-313, XP002432877 ISSN: 0340-6717
- LEUNG D Y M ET AL.,: "New insights into atopic dermatitis" JOURNAL OF CLINICAL INVESTIGATION, vol. 113, no. 5, March 2004 (2004-03), pages 651-657, XP002432878
- YAMANAKA K ET AL: "SKIN-SPECIFIC CASPASE-1-TRANSGENIC MICE SHOW CUTANEOUS APOPTOSIS AND PRE-ENDOTOXIN SHOCK CONDITION WITH A HIGH SERUM LEVEL OF IL-18" JOURNAL OF IMMUNOLOGY, THE WILLIAMS AND WILKINS CO. BALTIMORE, US, vol. 165, no. 2, 15 July 2000 (2000-07-15), pages 997-1003, XP002946985 ISSN: 0022-1767 -& MIZUTANI HITOSHI ET AL: "Animal models of psoriasis and pustular psoriasis." ARCHIVES OF DERMATOLOGICAL RESEARCH APR 2003, vol. 295 Suppl 1, April 2003 (2003-04), pages S67-S68, XP002432879 ISSN: 0340-3696
- WARNNISSORN P ET AL: "Tumour necrosis factor-related apoptosis-inducing ligand expression in atopic dermatitis." BRITISH JOURNAL OF DERMATOLOGY, vol. 148, no. 4, April 2003 (2003-04), pages 829-831, XP002432890 ISSN: 0007-0963
- NOMURA I ET AL: "Distinct patterns of gene expression in the skin lesions of atopic dermatitis and psoriasis: A gene microarray analysis" JOURNAL OF ALLERGY AND CLINICAL IMMUNOLOGY, MOSBY - YEARLY BOOK, INC, US, vol. 112, no. 6, December 2003 (2003-12), pages 1195-1202, XP002349917 ISSN: 0091-6749
- KOGA S ET AL: "A NOVEL TELOMERASE-SPECIFIC GENE THERAPY: GENE TRANSFER OF CASPASE-8 UTILIZING THE HUMAN TELOMERASE CATALYTIC SUBUNIT GENE PROMOTER" HUMAN GENE THERAPY, MARY ANN LIEBERT, NEW YORK ,NY, US, vol. 11, no. 10, 1 July 2000 (2000-07-01), pages 1397-1406, XP000979216 ISSN: 1043-0342
- VARFOLOMEEV E E ET AL: "Targeted disruption of the mouse caspase 8 gene ablates cell death induction by the TNF receptors, Fas/Apo1, and DR3 is lethal prenatally" IMMUNITY, CELL PRESS, US, vol. 9, August 1998 (1998-08), pages 267-276, XP002320053 ISSN: 1074-7613 cited in the application
- KANG TAE-BONG ET AL: "Caspase-8 serves both apoptotic and nonapoptotic roles." JOURNAL OF IMMUNOLOGY (BALTIMORE, MD. : 1950) 1 SEP 2004, vol. 173, no. 5, 1 September 2004 (2004-09-01), pages 2976-2984, XP002417111 ISSN: 0022-1767 cited in the application
- SALMENA L ET AL: "Caspase-8 deficiency in T cells leads to a lethal lymphoinfiltrative immune disorder" JOURNAL OF EXPERIMENTAL MEDICINE 19 SEP 2005 UNITED STATES, vol. 202, no. 6, 19 September 2005 (2005-09-19), pages 727-732, XP002417479 ISSN: 0022-1007
- BANNO T ET AL: "Pathway-specific profiling identifies the NF-[kappa]B-dependent tumor necrosis factor [alpha]-regulated genes in epidermal keratinocytes" JOURNAL OF BIOLOGICAL CHEMISTRY 13 MAY 2005 UNITED STATES, vol. 280, no. 19, 13 May 2005 (2005-05-13), pages 18973-18980, XP002432880 ISSN: 0021-9258
- WACHTER T ET AL: "cFLIPL inhibits tumor necrosis factor-related apoptosis-inducing ligand-mediated NF-[kappa]B activation at the death-inducing signaling complex in human keratinocytes" JOURNAL OF BIOLOGICAL CHEMISTRY 17 DEC 2004 UNITED STATES, vol. 279, no. 51, 17 December 2004 (2004-12-17), pages 52824-52834, XP002432881 ISSN: 0021-9258
- BROOKS A D ET AL: "Reduction of the antiapoptotic protein cFLIP enhances the susceptibility of human renal cancer cells to TRAIL apoptosis" CANCER IMMUNOLOGY, IMMUNOTHERAPY 2005 GERMANY, vol. 54, no. 5, May 2005 (2005-05), pages 499-505, XP002432882 ISSN: 0340-7004
- MCDONALD III E R ET AL: "Suppression of caspase-8- and -10-associated RING proteins results in sensitization to death ligands and inhibition of tumor cell growth" PROCEEDINGS OF THE NATIONAL ACADEMY OF SCIENCES OF THE UNITED STATES OF AMERICA 20 APR 2004 UNITED STATES, vol. 101, no. 16, 20 April 2004 (2004-04-20), pages 6170-6175, XP002432883 ISSN: 0027-8424 cited in the application
- FALLON FRIEDLANDER, S.: "Psoriasis and the Pediatric Practitioner: Emerging Therapies"[Online] 10 October 2002 (2002-10-10), pages 1-4, XP002433396 Retrieved from the Internet: URL:http://www.medscape.com/viewarticle/44 2550>
- DATABASE EMBL [Online] 17 March 2001 (2001-03-17), "Mus musculus interleukin 33, mRNA (cDNA clone MGC:6424 IMAGE:3593927), complete cds." XP002433061 retrieved from EBI accession no. EMBL:BC003847 Database accession no. BC003847 -& BAEKKEVOLD E S ET AL: "Molecular characterization of NF-HEV, a nuclear factor preferentially expressed in human high endothelial venules" AMERICAN JOURNAL OF PATHOLOGY, PHILADELPHIA, PA, US, vol. 163, no. 1, July 2003 (2003-07), pages 69-79, XP002290804 ISSN: 0002-9440
- DATABASE EMBL [Online] 9 February 2001 (2001-02-09), "Mus musculus adult male epididymis cDNA, RIKEN full-length enriched library, clone:9230117E20 product:hypothetical Kazal-type serine protease inhibitor domain containing protein, full insert sequence." XP002433062 retrieved from EBI accession no. EMBL:AK020352 Database accession no. AK020352
- DATABASE EMBL [Online] 26 January 2001 (2001-01-26), "Mus musculus putative small membrane protein NID67 mRNA, complete cds." XP002433063 retrieved from EBI accession no. EMBL:AF313412 Database accession no. AF313412
- AKDIS MÜBECCEL ET AL: "T helper (Th) 2 predominance in atopic diseases is due to preferential apoptosis of circulating memory/effector Th1 cells." THE FASEB JOURNAL : OFFICIAL PUBLICATION OF THE FEDERATION OF AMERICAN SOCIETIES FOR EXPERIMENTAL BIOLOGY JUN 2003, vol. 17, no. 9, June 2003 (2003-06), pages 1026-1035, XP002417112 ISSN: 1530-6860
- KRUIDERING M ET AL: "Caspase-8 in apoptosis: The beginning of 'the end'?" IUBMB LIFE 2000 UNITED STATES, vol. 50, no. 2, 2000, pages 85-90, XP008074187 ISSN: 1521-6543
- KLEMENT J F ET AL: "IkappaBalpha deficiency results in a sustained NF-kappaB response and severe widespread dermatitis in mice" MOLECULAR AND CELLULAR BIOLOGY, AMERICAN SOCIETY FOR MICROBIOLOGY, WASHINGTON, US, vol. 16, no. 5, May 1996 (1996-05), pages 2341-2349, XP002996623 ISSN: 0270-7306
- VASSINA EKATHERINA ET AL: "Increased expression and a potential anti-inflammatory role of TRAIL in atopic dermatitis." THE JOURNAL OF INVESTIGATIVE DERMATOLOGY OCT 2005, vol. 125, no. 4, 31 October 2005 (2005-10-31), pages 746-752, XP002432891 ISSN: 0022-202X
- WALLACH, D ET EAL.,: "Regulation of cell-death and immune defence by receptors of the TNF-NGF family" LIFE SCIENCES, [Online] 2006, pages 1-3, XP002432893 Retrieved from the Internet: URL:http://www.weizmann.ac.il/Biology/open _day_2006/book/Abstracts/David_Wallach.pdf >
- SCHMITZ J ET AL: "IL-33, an interleukin-1-like cytokine that signals via the IL-1 receptor-related protein ST2 and induces T helper type 2-associated cytokines" IMMUNITY 2005 UNITED STATES, vol. 23, no. 5, November 2005 (2005-11), pages 479-490, XP002432892 ISSN: 1074-7613
- ANONYMOUS: "Enbrel" INTERNET CITATION, [Online] pages 1-8, XP002433414 Retrieved from the Internet: URL:http://www.rxlist.com/cgi/generic/etan ercept_ad.htm> [retrieved on 2007-05-14]
- CHUN H J ET AL: "Pleiotropic defects in lymphocytes activation caused by caspase-8 mutations and to human immunodeficiency", NATURE, NATURE PUBLISHING GROUP, UNITED KINGDOM, vol. 419, 26 September 2002 (2002-09-26), pages 395-399, XP003003674, ISSN: 0028-0836, DOI: 10.1038/NATURE01063

## Description

### FIELD OF THE INVENTION

The invention relates to the effect of caspase-8 activity in the pathology or course of a skin disease, disorder or condition.

### BACKGROUND OF THE INVENTION

Members of the caspase cysteine protease family can be activated by a variety of death inducing agents to cleave a set of death-related cellular proteins and thus initiate programmed cell death (or apoptosis). Different caspases serve distinct roles in this process. Some caspases named "effector caspases" exert most of the cleavage of death-related substrates. Other caspases named "initiator caspases" serve to activate the effector caspases following their own activation by distinct death inducers with which they interact through specific N-terminal recognition sequences. Growing evidence indicates that in various cells, including lymphocytes, hematopoietic progenitors, endothelial cells and others, caspases also participate in vital functions (Kang et al., 2004). So far, there is still very little knowledge of the relative contribution of the different caspases to these non-apoptotic functions or to the mechanisms by which the caspases mediate these non-apoptotic functions. It is not clear what are the mechanisms that define whether caspase activation at a given situation will lead to death or promote vital functions. Some studies suggested that, unlike the induction of death, the non-apoptotic functions of the caspases do not involve their enzymatic function, but rather involves a role of the caspases as adapter proteins or allosteric regulators of other signaling proteins (Chaudhary et al., 2000). In contrast, other studies suggested that the enzymatic activity of the caspases does contribute to their non-apoptotic functions (Hasegawa et al., 2005).

As the body's largest and most environment-exposed surface, the skin has a central role in host defense. The skin consists of three layers: epidermis, dermis and hypodermis. Epidermis, the outermost layer of the skin, is made up of stratified squamous epithelium and is composed of the basal, spinous, granular and cornified layers (Fuchs and Segre, 2000, Lavker and Sun, 2000). The epidermal keratinocytes of the basal layers have the proliferative capacity and constitute a pool of epidermal stem cells. The transient amplifying cells are those stem cells that undergo 3∼5 more cycles of division. They are then destined to move upwards from the basal layers to undergo terminal differentiation (Potten, 1981, Adams and Watt, 1990).

The growth and differentiation of keratinocytes is regulated by many growth factors and cytokines including EGF, transforming-growth factor (TGF)-α, heparin binding EGF-like factor (HB-EGF), amphiregulin, keratinocyte growth factor (KGF), TGF-β, insulin-like growth factor, platelet derived growth factor (PDGF), hepatocyte growth factor (HGF), IL-6, IL-1 and TNF-α (Bennett and Schultz, 1993, Peus and Pittelkow, 1996, Martin, 1997).

Atopic dermatitis (AD) is a chronic, inflammatory skin disease that may occur at any age and is very common in children. AD has been reported to affect more than 10% of children and 1-3% of adults.

AD is associated with cutaneous hyper-reactivity to environmental triggers that are innocuous to normal non-atopic individuals. So far, no objective laboratory test for diagnosis of AD exists, and diagnosis is based on clinical observations, which may include pruritus, facial, and extensor eczema in infants and children, flexural eczema in adults, and chronicity of the dermatitis (Leung et al. 2004). Diagnosis of AD is based also on the distribution and duration of lesions and often on a family history of atopic disorders and the presence of lichenification (MERK manual online). Because atopic dermatitis is often hard to differentiate from seborrheic dermatitis in infants or from contact dermatitis at any age, the physician should examine the patient several times before making a definitive diagnosis.

Skin hydration, avoidance of irritants, antihistamines, topical corticosteroids and newer topical immunomodulators are the mainstay of therapy for AD. However, AD is usually refractory to treatment and the local and systemic side effects of topical steroids are widely recognized.

An understanding of the immunologic basis of AD is necessary for the development of novel approaches to treating this disease. AD is associated with diseases and conditions characterized by elevated serum IgE levels and peripheral eosinophilia such as allergic rhinitis, asthma and food allergy and the majority of patients afflicted with AD eventually develop allergic rhinitis or asthma.

A recent review by Leung (2004) summarizes the immunologic characteristics of AD. For example, most AD patients have elevated numbers of circulating eosinophils and increased immunoglobulin E (IgE) levels, which are caused by T-cell dysfunction. An increased frequency of Th2 cells that produce increased IL-4, IL-5 and IL-13 has been demonstrated in the peripheral blood of patients with AD. Factors contributing to Th2 cell development in AD include cytokines, genetic differences in cytokine (IL-4) production, pharmacologic factors (monocytes with increase CAMP phosphodiesterase activity) and antigen presenting cells (increased IgE-bearing Langerhans' cells with a role in cutaneous allergen presentation to Th2 cells).

IL-4 and IL-13 are the only cytokines that promote an increase in IgE production at the level of germline transcription. IL-5 induces eosinophilopoiesis, activation and chemotaxis. Eosinophils secrete cytokines and mediators that injure tissue via reactive 02 intermediates and the release of toxic granule proteins. Eosinophil granule proteins are increased in AD sera and correlate with disease activity.

Psoriasis is a common chronic, recurrent disease characterized by dry, well-circumscribed, silvery, scaling papules and plaques of various sizes (reviewed in the Merk manual online).

Psoriasis varies in severity from one or two lesions to widespread dermatosis, sometimes associated with disabling arthritis or exfoliation. The cause of psoriasis is unknown, but the thick scaling has traditionally been attributed to increased epidermal cell proliferation and concomitant dermal inflammation. The response of psoriasis to the immunosuppressive drug cyclosporine suggests that the primary pathogenetic factor may be immunologic. About 2 to 4% of whites and far fewer blacks are affected. Onset of psoriasis is usually between ages 10 and 40, but no age is exempt. A family history of psoriasis is common.

Onset of psoriasis is usually gradual. The typical course is one of chronic remissions and recurrences (or occasionally acute exacerbations) that vary in frequency and duration. Factors precipitating psoriatic flares include local trauma (in the Koebner's phenomenon, lesions appear at sites of trauma) and, occasionally, irritation (variants of Koebner's phenomenon), severe sunburn, viremia, allergic drug reactions, topical and systemic drugs (e.g., chloroquine antimalarial therapy, lithium, -blockers, interferon-), and withdrawal of systemic corticosteroids. Some patients (especially children) may have psoriatic eruptions after an acute group A - hemolytic streptococcal URI.

Psoriasis characteristically involves the scalp (including the postauricular regions), the extensor surface of the extremities (particularly elbows and knees), the sacral area, buttocks, and penis. The nails, eyebrows, axillae, umbilicus, or anogenital region may also be affected. Occasionally the disease is generalized.

Typical lesions are sharply demarcated, variously pruritic, ovoid or circinate erythematous papules or plaques covered with overlapping thick silvery micaceous or slightly opalescent shiny scales. Papules sometimes extend and coalesce to produce large plaques in annular and gyrate patterns, but this phenomenon is more common in cutaneous T-cell lymphoma. The lesions heal without scarring, and hair growth is usually unaltered. Nail involvement occurs in 30 to 50% of patients and may clinically resemble a fungal infection, with stippling, pitting, fraying, discoloration or separation of the distal and lateral margins of the nail plate (onycholysis), and thickening, with hyperkeratotic debris under the nail plate.

Erythrodermic psoriasis (exfoliative psoriatic dermatitis) may be refractory to therapy. The entire cutaneous surface is red and covered with fine scales; typical psoriatic lesions may be obscured or absent. It may lead to general debility and a need for hospitalization.

Pustular psoriasis is characterized by sterile pustules and may be generalized (von Zumbusch type) or localized to the palms and soles (Barber's psoriasis).

Psoriasis may be confused with seborrheic dermatitis, squamous cell carcinoma in situ (Bowen's disease, especially when on the trunk), secondary syphilis, dermatophyte infections, cutaneous lupus erythematosus, eczema, lichen planus, pityriasis rosea, or localized dermatitis caused by scratching (lichen simplex chronicus). However, diagnosis by inspection is rarely difficult; e.g., well-defined, dry, heaped-up psoriatic lesions with large silvery scales are usually distinguishable from the diffuse, greasy, yellowish scaling of seborrheic dermatitis.

Although biopsy findings of typical lesions are generally characteristic, atypical lesions have atypical features making biopsy less helpful; some other skin diseases may have psoriasiform histological features that may make microscopic diagnosis difficult or equivocal.

The prognosis of psoriasis depends on the extent and severity of the initial involvement. Usually the earlier the age of onset, the greater the severity. Acute attacks usually clear, but permanent remission is rare.

WO 98/37901 relates to the therapeutic induction of apoptosis in activated inflammatory cells, or cells at a site of inflammation, by introducing into those cells a chimeric gene containing an apoptosis-inducing gene (AIG) driven by a promoter of an inducible gene activated in inflammation and a promoter enhancer such that the inflammatory cells are targeted. In one embodiment, the chimeric gene comprises at least one TNF alpha promoter enhancer attached to a functional copy of a minimal TNF alpha promoter and further attached to at least one copy of an apoptosis-inducing gene, wherein expression of the gene is driven by the TNF alpha promoter. Attachment can be direct, distal, proximal or combinations thereof. Example apoptosis-inducing genes include caspase 3, caspase 4, caspase 5, Granzyme B. Advantageously, the TNFp-AIG chimeric gene is expressed in only those cells producing the inflammatory cytokine, TNF alpha. In addition, the TNFp-AIG chimeric gene also sequesters inducible TNFp transcription factors, thereby reducing endogenous production of TNF alpha. The disclosure also relates to methods of making and using self-regulated apoptosis chimeric genes and pharmaceutical compositions containing them for treating inflammatory diseases.

VARFOLOMEEV E E ET AL, discloses "Targeted disruption of the mouse caspase 8 gene ablates cell death induction by the TNF receptors, Fas/Apo1, and DR3 is lethal prenatally", IMMUNITY, CELL PRESS, US, (199808), vol. 9, ISSN 1074-7613, PAGE 267 - 276.

CHUN H J et al relates to "pleiotropic defects in lymphocytes activation caused by caspase-8 mutations and to human immunodeficiency", Nature, nature publishing group, United Kingdom, vol.419, 2002-09-26, pages 395-399.

WO 2005/001052 relates, at least in part, to methods of modulating apoptosis in cells, comprising contacting the cell with a compound that modulates the expression, post-translational modification, or activity of Ian4, leucine-rich protein of 130kD, GRP78, GRP94, or hsp60. The subject methods can be used to treat a variety of different disorders, for example, autoimmune disease and neoplasia. The disclosure also pertains to methods for identifying compounds that modulates the activity of Ian-4, leucine-rich protein of 130kD, GRP78, GRP94, or hsp60.

### SUMMARY OF THE INVENTION

The present invention relates to the use of an agent selected from caspase-8 or a fragment of caspase-8 that retains biological activity of caspase-8, in the manufacture of a medicament for the treatment or prevention of an inflammatory skin disease, disorder or condition.

The present invention relates also to an agent selected from caspase-8 or a fragment of caspase-8 that retains the biological activity of caspase-8, for use in the treatment or prevention of an inflammatory skin disease, disorder or condition.

Preferably the inflammatory skin disease is atopic dermatitis or psoriasis.

The present invention relates also to a method for diagnosing in an individual a skin disease, disorder or condition associated with caspase-8 deficiency in the skin or the predisposition to develop said skin disease, disorder or condition, comprising, measuring in a sample of skin of the tested individual and in a sample of skin of at least one healthy control individual the expression level or activity of caspase-8 and/or detecting aberrations in nucleic acid encoding caspase-8 in a sample of skin from the tested individual, wherein detection of a decrease of caspase-8 activity or expression level in the sample of the tested individual as compared to the sample of said at least one healthy control individual, and/or detection of aberrations in nucleic acid encoding caspase-8 in the tested individual is indicative of said skin disease, disorder or condition, or of a predisposition to develop said skin disease, disorder or condition in the tested individual.

Preferably the method for diagnosing in an individual a skin disease, disorder or condition is associated with caspase-8 deficiency in epithelial keratinocytes or the predisposition to develop said skin disease, disorder or condition.

The present invention relates also to a method for generating a non-human animal model of an inflammatory skin disease, disorder or condition, comprising developing a non-human animal arrested in keratinocyte caspase-8 expression level or activity.

Preferably the arrest of caspase-8 expression level is effected by knocking out caspase-8 in epithelial keratinocyte of the non-human animal.

Preferably the arrest of caspase-8 activity is effected by inducing transgenic expression of caspase-8 mutant lacking enzymatic activity in the non-human animal.

Preferably the transgenic caspase-8 mutant is a bacterial artificial chromosome with mutant caspase-8 lacking enzymatic activity, in which the Cys at residue 362 has been replaced for Ser.

Preferably the non-human animal is mouse.

The present invention relates also to a method for identifying a target protein involved in the pathology or course of a skin disease, disorder or condition whose level of expression or activity is normally regulated by caspase-8 activity in the skin comprising the steps of: comparing the profile of gene expression in a sample of skin comprising epithelial keratinocytes arrested in caspase-8 expression level or activity to the profile of gene expression in a sample of skin comprising epithelial keratinocytes having normal caspase-8 expression level or activity, wherein a gene whose expression is downregulated or upregulated in the sample comprising epithelial keratinocytes arrested in caspase-8 expression level or activity encodes a candidate target protein involved in the pathology or course of said skin disease, disorder or condition.

Preferably the skin disease, disorder or condition is an inflammatory skin disease, disorder or condition.

Preferably the disease is atopic dermatitis or psoriasis.

The present invention relates also to a method of screening of a candidate compound for treating or preventing a skin inflammatory disease, disorder or condition comprising, providing a culture of cells comprising keratinocytes arrested in caspase-8 expression level or activity, introducing a test compound in said cells, inducing differentiation of the cells by increasing the calcium concentration in the culture medium of the cells, measuring the expression level of a differentiation marker of the skin and/or p21ARF in the presence or absence of the test compound, wherein increase in the expression level of a differentiation marker of the skin and/or decrease of p21ARF in the presence of the test compound is indicative that the test compound is a candidate compound for treating or preventing said disease, disorder or condition.

Preferably the differentiation marker is selected from keratin 1, filagrin, and loricrin.

The present invention relates also to a method for testing the efficacy of a candidate compound for treating a skin inflammatory disease, disorder or condition, comprising administering to a non-human animal model generated according to any one of claims 6 to 10 the candidate test compound and assessing prevention or reduction of skin pathology in said animal model.

### BRIEF DESCRIPTION OF THE FIGURES

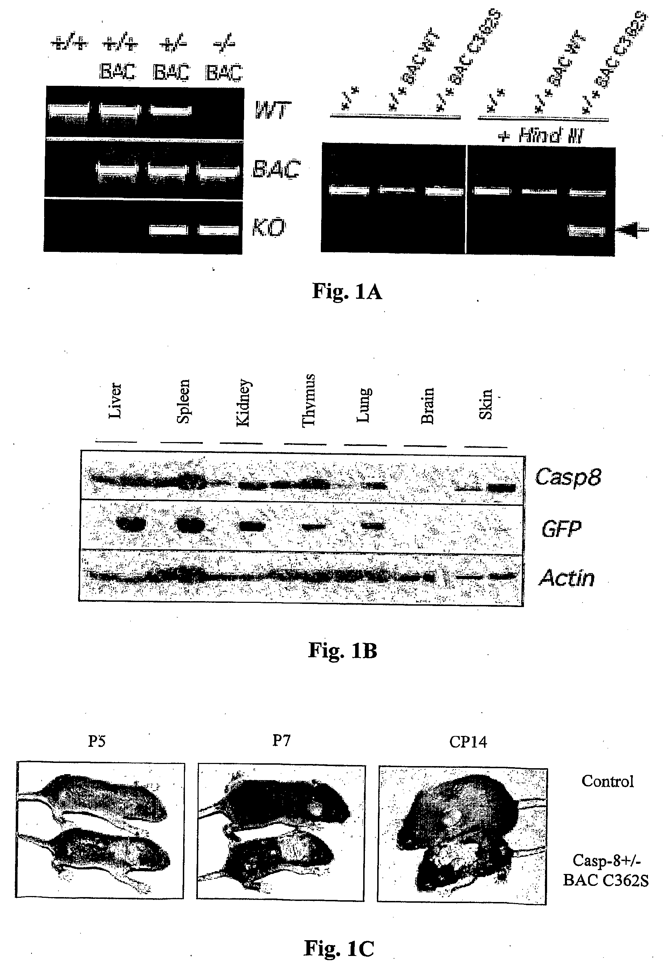

**Figs. 1A-E** show that transgenic mice expressing enzymatically inactive caspase-8 develop inflammatory skin disease. **(A)** The left panel shows PCR genotyping in samples from (from left to right) control mice having only endogenous caspase-8 gene (+/+), transgenic mice having endogenous caspase-8 gene and caspase-8 from bacterial artificial chromosome (+/+ BAC), transgenic mouse having BAC caspase-8 in a partial-knockout caspase-8 background (only knockout in one allele of caspase-8) (+/- BAC), and transgenic mouse having only BAC caspase-8 in a full-knockout caspase-8 background (-/- BAC). Unlike caspase-8 full-knockout mice, which die in *uteri,* -/- BAC mice do survive. The first half of the right panel shows PCR genotyping (from left to right) of samples from +/+, +/+ BAC, and +/+ BAC C362S (having BAC with mutant caspase-8 lacking enzymatic activity, in which the Cys at residue 362 has been replaced for Ser). The second half of the right panel shows the same samples and in the same order as in the first half but which were restricted with Hind III in order to distinguishes mutant BAC-C362S from the wild-type caspase-8. Hind III does cleave BAC-C362S mutant caspase-8 (arrow), but it does not cleave endogenous or BAC WT caspase-8. **(B)** shows a Western blot analysis of caspase-8 expression in samples from (left to right) liver, spleen, kidney, thymus, lung, brain, and skin of control mouse (+/+) or transgenic mouse expressing, in addition to the endogenous caspase-8, transgenic BAC caspase-8 (+/+ BAC) both controlled by the cognate caspase-8 promoter. The upper panel shows the levels of total caspase-8 (endogenous and transgenic). The first lane shows caspase-8 levels in a given tissue of a +/+ mice and the second lane shows caspase-8 of the same tissue of a +/+BAC mice having caspase-8 expressed from endogenous and transgenic origin. The middle panel shows GFP, which is present only in transgenic mice, and its level correlates with the levels of expression of transgenic caspase-8 (caspase-8 and GFP expression are both under the control of the cognate caspase-8 promoter). The lower panel shows the level of actin as control of amount of protein loaded in the different lanes (about 25 µg tissue lisate/lane). The observed pattern of total caspase-8 and GFP expression in control and transgenic mice indicates that transgenic caspase 8 alike endogenous caspase-8 is highly expressed in liver, spleen, kidney, thymus and lung, but is not expressed in the brain. It was also observed that both, endogenous and transgenic caspase-8 are expressed in the skin. Thus, the cognate caspase-8-promoter allows similar tissue specific expression of both endogenous and transgenic caspase-8. (C) Shows the general appearance of transgenic mice expressing mutant caspase-8 (BAC C362S) in a partial-knockout caspase-8 background (Casp8+/-) at postnatal day 5 (P5) 7 (P7), and 14 (P14). The result in the figure shows that mice expressing mutant caspase-8 defective in enzymatic activity, even in the presence of endogenous caspase-8, develop a skin disease, which progresses with time. **(D)** shows histology analysis of skin sections from transgenic mice bearing BAC C362S and control mice at P0 and P7 stained with haematoxilin/eosin. The result in the figure show that in control (WT) mice the epidermal layer at P0 (within 24 hours postnatal) is thicker than that at P7. At P0, no differences were apparent in the histological pattern of transgenic mice and control mice. Differences were observed at P7, which were manifested by cell infiltration and thickened epidermis in transgenic mice expressing the mutant caspase-8. (E) shows the schematic illustration of BAC modifications used in the above experiments (see Material and Methods).

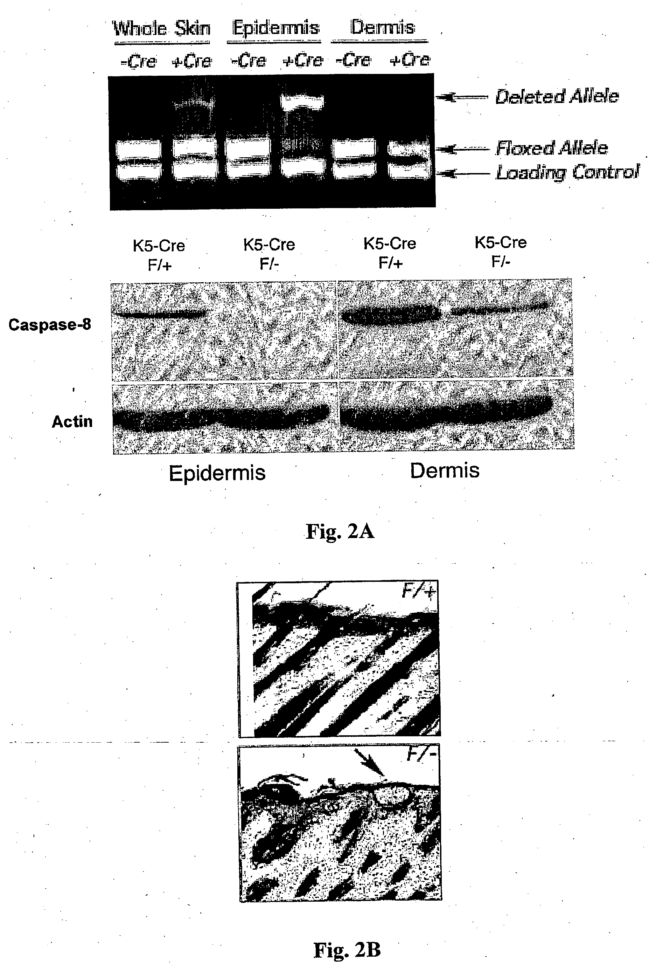

**Figs. 2A-D** show that mice bearing epidermis-specific-knockout of caspase-8 develop inflammatory skin disease. **(A)** Upper panel shows assessment of caspase-8 deletion in samples from (left to right) whole skin, epidermis or dermis of Casp-8^{f1/-} Cre- or Casp-8^{f1/-} Cre+ mice by genomic PCR. Lower panel shows assessment of caspase-8 deletion in samples from epidermis or dermis of K5 Casp-8^{f1/-} Cre- or K5 Casp-8^{f1/-} Cre+ mice by Western blot analysis. Extracts of epidermis or dermis was detected by western blotting with anti-caspase-8 (3B10). The results in the figure show that crossing mice in which part of one of the caspase-8 alleles was flanked by loxP sites and the other allele was deleted (Casp-8^{f1/-}) with transgenic mouse line that express Cre under control of keratinocyte-specific Keratin-5 promoter resulted in effective and exclusive deletion of caspase-8 gene in the epidermis **(B)** shows analysis of β-galactosidase expression (a Cre reporter in ROSA26 mice) in tissue sections from the skin of P7 ROSA26Casp-8^{f1/+}K5-Cre (upper panel) and ROSA26Casp-8^{f1/-}K5-Cre mice (lower panel), confirming specific Cre-induced expression (darker stain) in the epidermis and hair follicles of mice. **(C)** shows the phenotype of Casp-8^{f1/-}K5-Cre mice on a wild-type (upper panel) and on TNF-/- knockout background (lower panel) at different times after birth. The results obtained show that in mice deficient in TNF, the onset of the skin disease mediated by caspase-8 deficiency is not prevented but delayed. **(D)** shows the survival curves of K5F/- mice (Casp-8^{f1-}KS-Cre mice), and TNF^{-/-}K5FI/- mice. The result in the figure reveals that TNF deficiency delays death of the epidermis-specific caspase-8 -knockout mice.

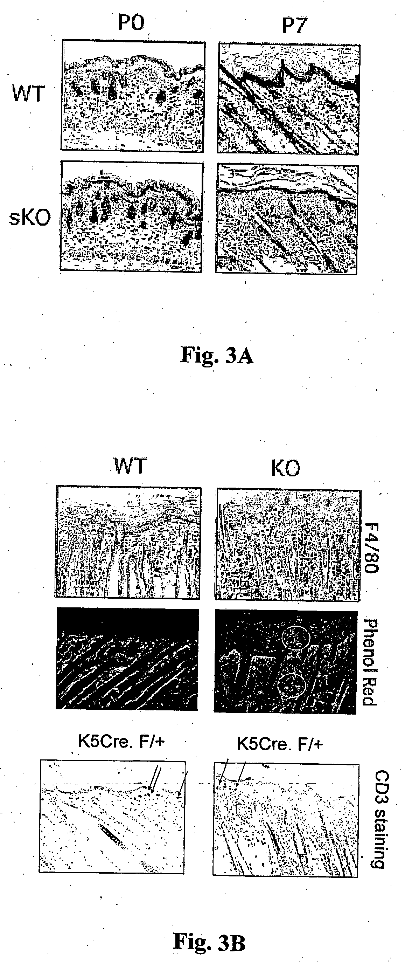

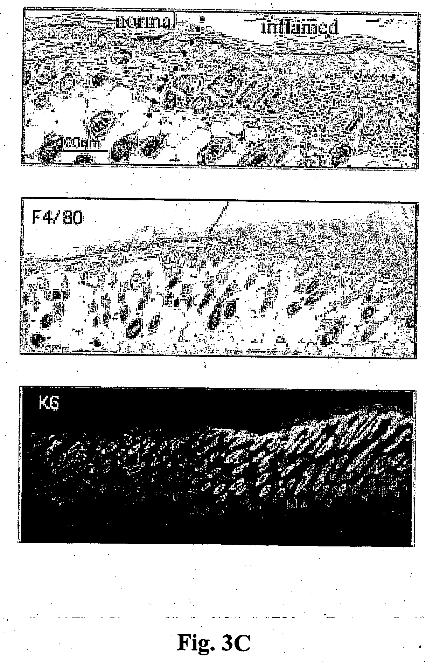

**Figs. 3A-C** show histology analysis and assessment of skin inflammation in Casp-8^{f1/-}K5-Cre. **(A)** shows hematoxylin/eosin staining of WT mice (upper panel) and mice deficient in caspase-8 targeted to the epidermis (sKO, lower panel) at P0 (left) and at P7 (right side). At P0 no difference were found in WT and caspase-8 deficient mice. At P7 increased cellularity was found in the dermis of Casp-8^{f1/-}K5-Cre P7 mice. **(B)** shows staining for macrophages (F4/80 antibody), eosinophils (phenol-red uptake) and T lymphocytes (anti-CD3 antibody) in skin sections of WT mice or Casp-8^{f1/}-K5-Cre mice at P7. The results in the figure demonstrate infiltration of macrophages and eosinophiles but not of T lymphocytes in the skin of Casp-8^{f1/}-K5-Cre mice. Identical staining pattern was observed on the Casp-8^{f1/-}K5-Cre in TNFR1-/- background (not shown). Of note is that eosinophils also accumulate within pustules in the epidermis of the Casp-8^{f1/-}K5-Cre mice. These results indicate that the skin disease developing in skin targeted caspase-8 deficient mice is inflammatory. (C) shows restriction of the inflammation to the regions of the epidermal lesions in P3 mice. The general increase of dermal cellularity (top), accumulation of F4/80-positive cells (middle), and staining with anti-keratin 6, a marker for epidermal activation (bottom) are shown to be restricted to the region of epidermal thickening (delineated with a broken lane).

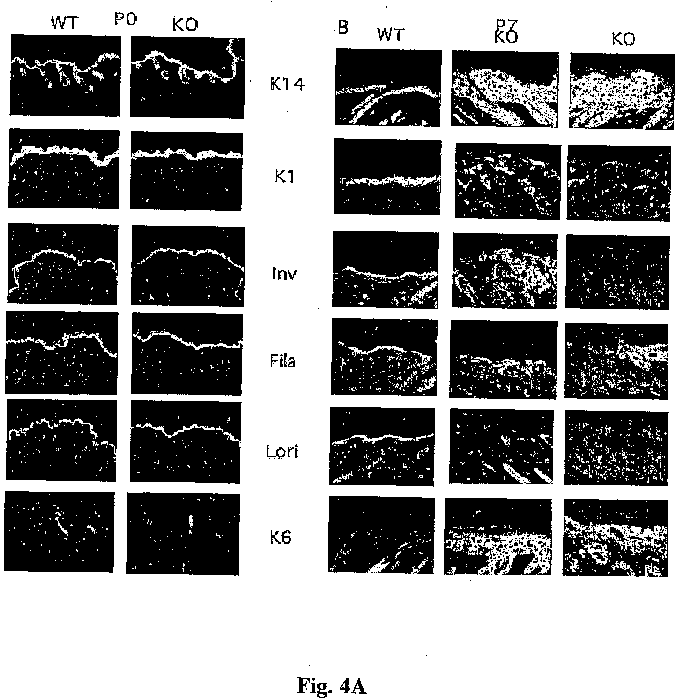

**Figs. 4A-C** show immunohistochemical analysis of the caspase-8-deficient epidermis. **(A)** the right panel shows imnunostaining of sections of skin of WT mice (left), of skin lesion of a Casp-8^{f1/-}K5-Cre mice (middle) and of severe skin lesion of the same Casp-8^{f1/-}K5-Cre mice (right) at P7. Immunostaining of the basal layer maker, K14, shows extension of the expression of this protein throughout the Casp-8^{f1/-}K5-Cre epidermis; staining for the differentiation markers K1 (granular layer), Involucrin (cornified layer) Fligarin (cornified layer) and Loricrin (cornified layer), shows disorganized localization, marker decrease and, eventually, complete absence of these proteins in the more severe Casp-8^{f1/-}K5-Cre lesion areas at P7. Also shown is the massive staining with anti-K6, a general marker for epidermal activation. None of these changes could be discerned at P0 (left panel), nor in P1 (not shown). **(B)** shows immunostaining for phospho-cJun of skin sections of WT and KO mice at P0 (left panel) and P7 (right panel). No changes were observed on the different skin sections tested. **(C)** TUNEL staining for assessing apoptosis (right), and Ki67 staining for determining cell proliferation (left) in the Casp-8^{f1/+}-K5-Cre and Casp-8^{f1/-}K5-Cre epidermis at P7. The results show increased apoptosis (indicated by arrows) and increased cell proliferation in the skin sample of Casp-8^{f1/-} K5-Cre mice.

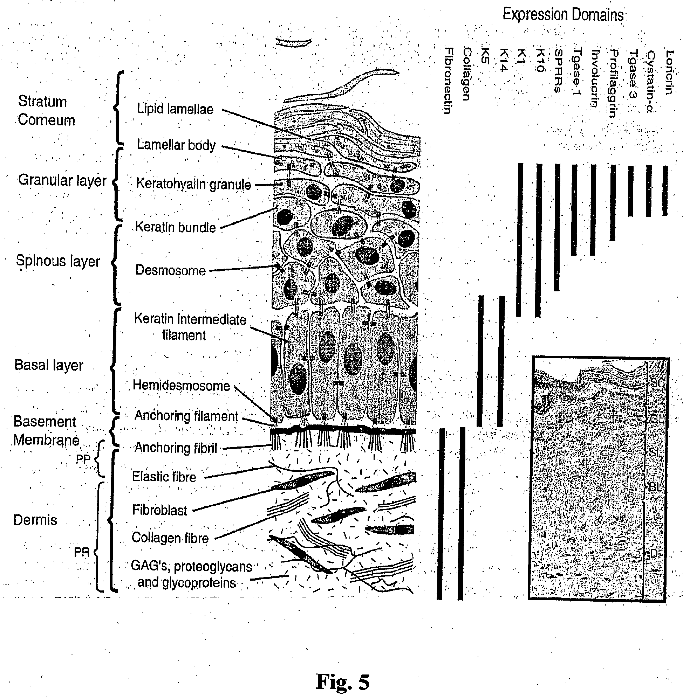

**Fig. 5** shows a schematic illustration of the layers of the skin and specific protein expression in each skin layer. In the skin there are two big layers (dermis and epidermis). Epidermis consists of 4 layers- basal, spinous (suprabasal), Granular and stratum corneum (comified) layer. Each layer expresses different proteins.

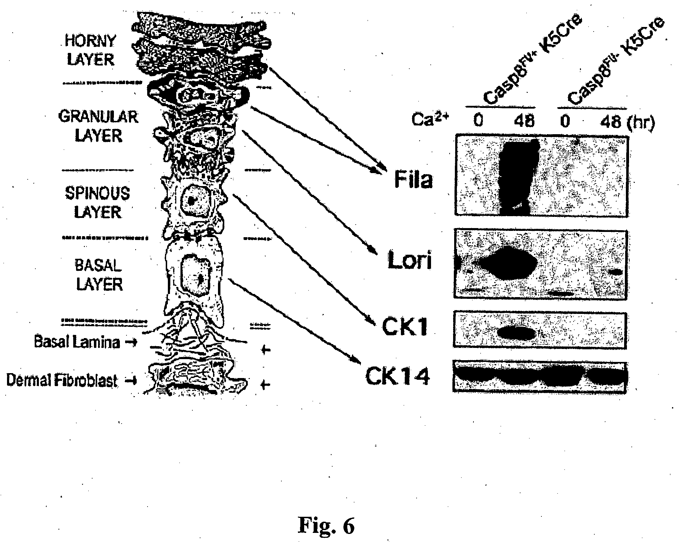

**Fig. 6** shows Western blot analysis of the total protein from cultured keratinocytes derived from P3 caspase-8^{f1/+} K5-Cre and caspase-8^{f1/-}K5-Cre mice lysed at the indicated times (hr) following 0.12 mM calcium exposure. Filaggrin (Fila) and loricrin (Lori) were used as the terminal differentiation markers and keratin 1 (CK1) as the intermediate differentiation marker. Keratin 14 (CK14) provided as a loading control. The results in the figure indicate that unlike cells expressing normal caspase-8, cells deficient in caspase-8 do not show Ca2+ induced differentiation markers fila, lori, and CK1.

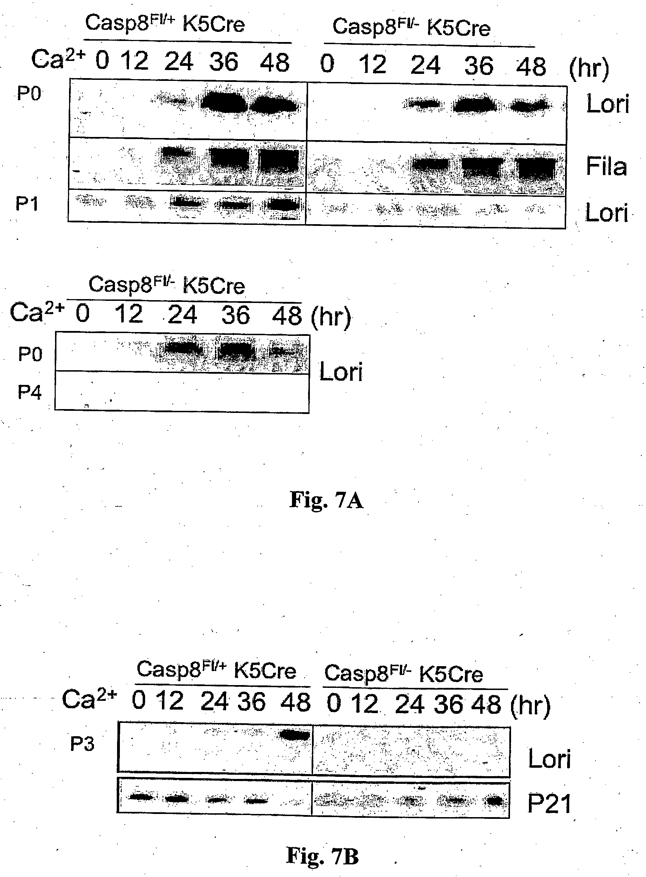

**Figs. 7A-B** show expression of differentiation markers in attached keratinocytes cultured in the presence of 0.12 mM calcium for the indicated times (hr). Total cell extracts (15µg) from attached keratinocytes were analyzed by Western blot analysis. **(A)** shows defective expression of the differentiation markers in cultured keratinocytes from caspase-8^{f1/+} K5-Cre and caspase-8^{f1/-} K5-Cre mice at P1, P4, but not at P0, as revealed by Western blot with antibodies against loricrin and filaggrin; **(B)** Lack of loricrin expression and proper p21 degradation upon calcium stimulation in the caspase-8^{f1/+ K5}-Cre keratinocyte of a P3 culture.

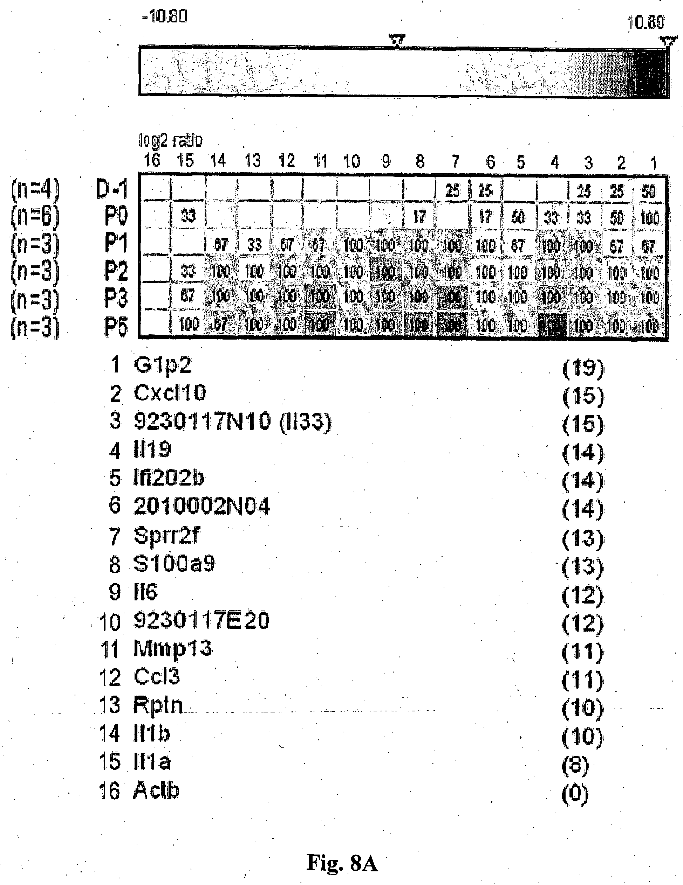

**Figs. 8A-8B** shows upregulation of gene expression in caspase-8^{f1/-} K5-Cre by RT-PCR (A) RNA was isolated from the epidermis of caspase-8-deficient or wild type animals before birth (D-1), immediately after birth (P0) or at days 1 (P1), 2, (P2), 3 (P3) or 5 (P5) after birth and used to determine expression of 16 genes G1p2, CXC110, 9230117N10Rik (IL33), IL-19, 1f1202b, 2010002N04Rik (SEQ ID NO: 3), Sprr2f, S100a9, IL-6, 9230117E20Rik (SEQ ID NO: 2), MMP13, Ccl3, Rptn, IL1b, IL1a and actin beta (as the control) by real time PCR. The numbers in parentheses, below the heatmap refer to the total number of caspase-8-deficient animals (out of a total of 22 tested) that show a significant increase in mRNA expression of each indicated gene. The percentages of individual caspase-8-deficient animals that show significant upregulation of mRNAs are depicted within the rectangles of the heatmap. (B) shows the same analysis as in (A) performed with samples from caspase-8-deficient animals in a TNF-/- background.

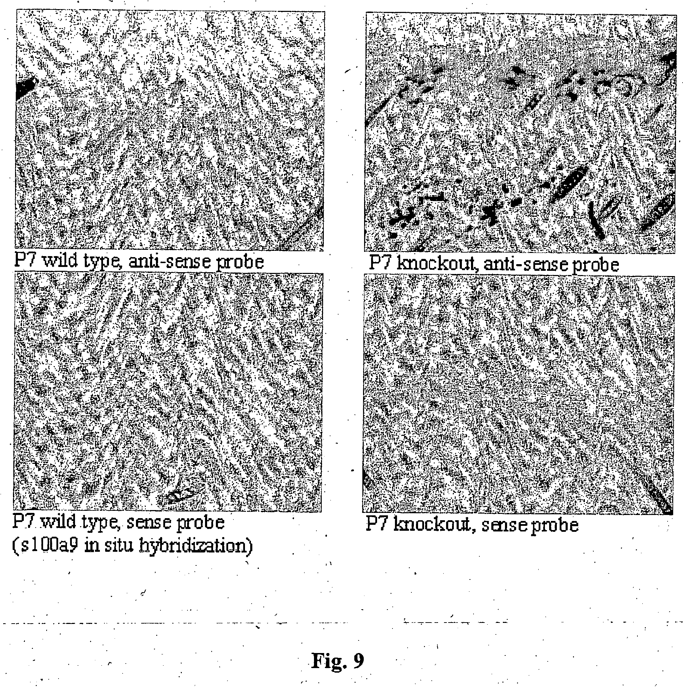

**Figs. 9** shows by in situ hybridization analysis that S100A9 mRNA expression is dramatically upregulated in lesion of skin of knockout mice. Detection was carried out with a Dig-labeled s100a9 probe

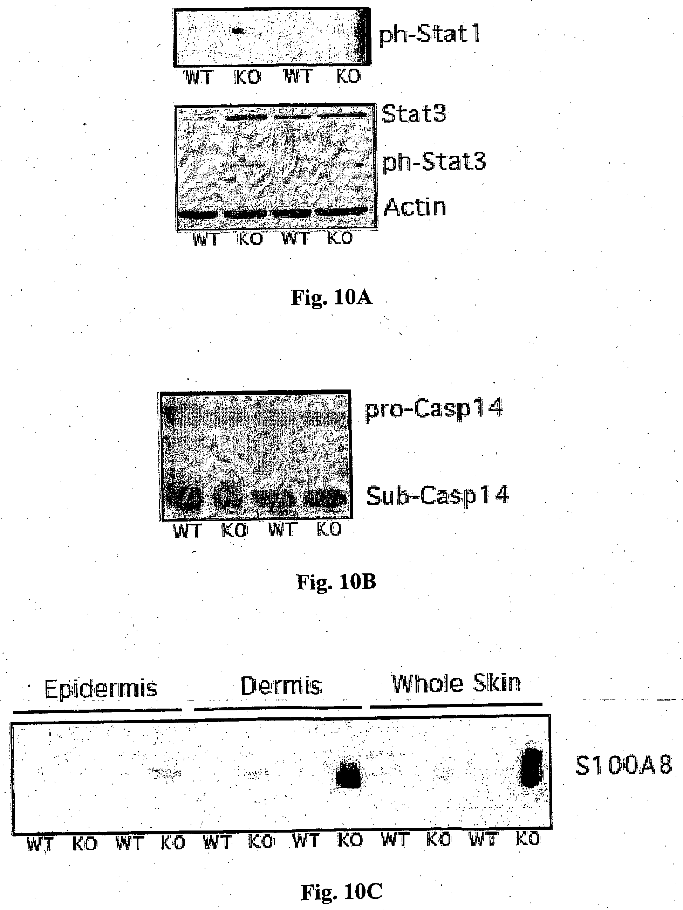

**Figs. 10A-10C** (A) Shows activation of stat1 and stat3 in caspase-8 deficient skin. Western blotting analysis of phospho-stat1, phospho-3 and total stat3 was carried out on samples of the total protein extracted from the epidermis of caspase-8-(KO) and caspase-8 +(WT) mice (P4, two individual mice). The level of actin in the same samples was assessed as a loading control.(B) Shows activation of caspase-14. Western blotting analysis were carried out in order to show extent of the processing and activation of caspase-14 carried out on samples of the total protein extracted from the epidermis of caspase-8-(KO) and caspase-8 +(WT) mice (P4, two individual mice).(C) Shows differential expression of s100a8 protein in caspase-8 deficient skin. S100a8 level was determined by western blot analysis of samples of epidermis, dermis and whole skin extracted from caspase-8- (KO) and caspase-8 + (WT) mice (P4, two individual mice).

### DETAILED DESCRIPTION OF THE INVENTION

It has been found according to the present invention, that deficiency of caspase-8 in the skin is associated with the development of an inflammatory skin disease. We found that transgenic expression in mice of an enzymatically inactive caspase-8 (BAC-C362S), which apparently inhibits the activity of the endogenous caspase-8, or that specific deletion of capsase-8 in keratinocytes in mice, induces inflammatory skin hyperplasia. We also found that arrest of keratinocyte differentiation is involved in the pathology induced by deficiency of caspase-8 activity. Notably, the epidermis of mice having specific deletion of caspase-8 in keratinocytes (Casp-8^{f1/-}K5-Cre mice) or of transgenic mice expressing enzymatically inactive caspase-8 (Casp-8^{-/+}/BAC-C362S mice) displayed similar features to epidermis of atopic dermatitis (AD) patients such as a local elevation of eosinophiles at the damaged site and induction of TH2 response.

The findings according to the invention, that development of a skin inflammatory disease, disorder or condition, is caused by caspase-8 deficiency in the skin, paves the way to the development of new therapies to treat or prevent said disease disorder or condition.

Thus, the invention relates to the prevention or treatment of an inflammatory skin disease, disorder or condition, by modulating a protein that is normally regulated by caspase-8 in the skin or by increasing caspase-8 in the skin. Thus, in one aspect, the present invention relates to the use of an agent selected from, caspase-8 or a fragment thereof , in the manufacture of a medicament for the treatment or prevention of an inflammatory skin disease, disorder or condition.

US Patents 6,399,327 and 6,586,571, of the present applicant, disclose inter alia the amino acid sequence of caspase-8 (or as previously designated, MACH) and the nucleotide sequence encoding caspase-8, assays of protease (or enzymatic) activity of caspase-8, and the binding of caspase-8 to the protein MORT-1 (or FADD).

A "fragment" according to the present invention may be an active fragment of caspase-8. The term fragment refers to any subset of the caspase-8 molecule, that is, a shorter peptide that retains the desired biological activity of caspase-8. Fragments may readily be prepared by removing amino acids from either end of caspase-8 and testing the resultant fragment for its enzymatic activity. Proteases may be used for removing one amino acid at a time from either the N-terminal or the C- terminal of a polypeptide are known, and thus determining fragments, which retain the desired biological activity, involves only routine experimentation and are described in US Patents 6,399,327 and 6,586,571, of the present applicant.

It should be understood that modified caspase-8 molecules having qualitatively the same biological activity as caspase-8 are encompassed herein by the invention. These modified caspase-8 include: (i) muteins, analogs in which one or more of the amino acid residues are deleted or replaced by different amino acid residues, and/or one or more amino acid residues are added, without changing considerably the activity of the resulting products as compared with the original protein, and obtained by known synthesis and/or site-directed mutagenesis techniques; (ii) functional derivatives, obtained by chemical substitution of functional groups in side chains of amino acid residues or at the N- and/or C-terminal groups, as long as they remain pharmaceutically acceptable, i.e., they do not destroy the activity of the protein. Such derivatives may, for example, include esters, amides and polyethylene glycol (PEG) side-chains; and (iii) salts, including both salts of carboxyl groups and acid addition salts of amino groups of the polypeptide.

A Polynucleotide encoding caspase-8 or a fragment thereof, or of an expression vector comprising said polynucleotide can be used in the manufacture of a medicament for the treatment or prevention of an inflammatory skin disease disorder or condition.

The term "nucleic acid molecule" or "polynucleotide" as used herein refers to a deoxyribonucleotide or ribonucleotide polymer in either single-stranded or double-stranded form, and, unless specifically indicated otherwise, encompasses polynucleotides containing known analogs of naturally occurring nucleotides that can function in a similar manner as naturally occurring nucleotides. It will be understood that when a nucleic acid molecule is represented by a DNA sequence, this also includes RNA molecules having the corresponding RNA sequence in which "U" (uridine) replaces "T" (thymidine).

The polynucleotides include also polynucleotides that comprise degenerate codons and/or which hybridize under highly stringent conditions to the complementary sequences of said polynucleotides.

The term "stringent conditions" refers to a temperature and ionic conditions used in a nucleic acid hybridization reaction (See Ausubel et al., Current Protocols in Molecular Biology, supra, Interscience, N.Y., §§6.3 and 6.4 (1987, 1992), and Sambrook et al. (Sambrook, J. C., Fritsch, E. F., and Maniatis, T. (1989) Molecular Cloning: A Laboratory Manual, Cold Spring Harbor Laboratory Press, Cold Spring Harbor, NY). Stringent conditions are sequence dependent and are different under different environmental parameters. Generally, stringent conditions are selected to be about 5 to 10 °C or to 20 °C lower than the thermal melting point (Tm) for the specific sequence at a defined ionic strength and pH. The Tm is the temperature, under defined ionic strength and pH, at which 50% of the target sequence hybridizes to a perfectly matched probe. Stringent conditions will be those in which the salt concentration is less than about 1.0 M sodium ion, typically about 0.01 to 1.0 M sodium ion concentration (or other salts) at pH 7.0 to 8.3 and the temperature is at least about 30 °C for short probes (for example, 10 to 50 nucleotides) and at least about 60 °C for long probes (for example, greater than 50 nucleotides). Stringent conditions may also be achieved with the addition of destabilizing agents such as formamide. Examples of stringent conditions include washing conditions 5°C to 10°C lower than the calculated Tm of the hybrid under study in, e.g., 2 x SSC and 0.5% SDS for 5 minutes, 2 x SSC and 0.1% SDS for 15 minutes; 0.1 x SSC and 0.5% SDS at 37°C for 30- 60 minutes and then, a 0.1 x SSC and 0.5% SDS at 68°C for 30-60 minutes.

The polynucleotides include also polynucleotides that comprise degenerate codons. Because of the degeneracy of the genetic code, a large number of functionally identical polynucleotides encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. It will also be recognized that each codon in a polynucleotide, except AUG, which is ordinarily the only codon for methionine, and UUG, which is ordinarily the only codon for tryptophan, can be modified to yield a functionally identical molecule by standard techniques.

Expression vectors are well known in the art and are described, for example in Current Protocols in Molecular Biology. Expression vectors can be a plasmid vector, viral vector, and the like. Generally, the vector contains a selectable marker independent of that encoded by a polynucleotide of the invention, and further can contain transcription regulatory element such as a promoter or polyadenylation signal sequence, or a translation regulatory element such as a ribosome binding site. A promoter sequence can provide tissue specific expression of a polynucleotide operatively linked thereto. In one alternative the promoter is active in skin cells. In another alternative, the promoter is active in epidermis cells.

Also is described the use of an activator of the level or activity of caspase-8 in a skin inflammatory disease, disorder or condition.

The term "activator of a protein" refers to any agent, such as a protein, nucleotide and small molecule, capable of up-regulating said protein production and/or action. For example, an activator of caspase-8 may be a molecule that act upstream of caspase-8 in the skin.

Examples of activators of caspase-8 include, but are not limited to, FADD, caspases that can cleave caspase-8, that is - caspase-6 and caspase-3 and, indirectly, the various death receptors of the TNF/NGF family. Depending on the exact cellular set up, cFLIP long may also serve as caspase-8 activator.

A vector for inducing and/or enhancing the endogenous production of caspase-8 is also contemplated as an activator of caspase-8 according to the invention. The vector may comprise regulatory sequences capable of enhancing the expression of caspase-8. Such regulatory sequences may be, for example, promoters or enhancers. The regulatory sequence may then be introduced into the right locus of the genome by homologous recombination, thus operably linking the regulatory sequence with the gene, the expression of which is required to be induced or enhanced. The technology is usually referred to as "endogenous gene activation" (EGA), and it is described, e.g., in WO 91/09955.

Also described is the use of an inhibitor of a natural inhibitor of caspase-8 activation in a skin inflammatory disease, disorder or condition.

The following are examples of natural inhibitors of caspase-8 activation, whose level or activity may be inhibited, which include but are not limited to, (i) cFLIP short (CASH beta) (ii) cFLIP long (CASH alpha). (iii) The caspases-8- and -10-associated RING proteins (CARPs, McDonald ER 3rd, El-Deiry WS, Proc Natl Acad Sci U S A. 2004 Apr 20; 101(16):6170-5).

The term "inhibitor " refers to any agent such as a protein (e.g. a specific antibody), nucleotide (e.g. a specific antisense and small Interfering RNAs [siRNA]) and a specific inhibitory small molecule capable of down-regulating the production and/or action of a protein in such a way that said protein production and/or action is attenuated, reduced, or partially, substantially or completely prevented or blocked.

For example, a specific siRNA can be used for post-transcriptional silencing of specific mRNA targets (Dorsett and Tuschl 2004). Target specificity in RNAi is achieved through RNA-RNA sequence recognition and base pairing. The siRNA consists of double stranded RNA, typically of 19-21 base pair long, with two nucleotides overhanging at each 3' end. For maximal stability, two 2' deoxynucleotides are used as 3' overhangs. Alternatively, 27-mer blunt-ended nucleotides may be used, as these have shown improved efficiency in gene silencing (Kim, Behlke et al. 2005). Transport of siRNA into cells may be enhanced by encapsulation into liposomes or by covalent coupling to highly lipophilic agents. Soutschek et al (2004).

Another example of an inhibitor of expression is a specific short hairpin RNA (shRNA). Designing and cloning strategies for constructing shRNA expression vectors are known in the art (McIntyre and Gregory et al BMC Biotechnology 2006, 6:1).

An inhibitor of the level or activity of a protein, which is normally downregulated by caspase-8 activity in the skin, can be used in the treatment or prevention of an inflammatory skin disease, disorder or condition.

A protein, which is normally downregulated by caspase-8 activity in the skin, can become upregulated when caspase-8 activity or level in the skin is arrested. The following are non limiting examples of proteins which are normally downregulated directly or indirectly by caspase-8 activity in the skin and that were found according to the present invention: ISG15 (G1p2), Cxcl10, 9230117N10Rik (IL33)-human orthologue is called C90rf26, IL-19, Sprr2f, S100a9, IL-6, MMP13, Ccl3, IL1b, and proteins of unknown function such as homologous human proteins to the proteins encoded by 9230117E20Rik (SEQ ID NO: 2), 2010002N04Rik (SEQ ID NO:3).

Alternatively, an inhibitor capable of inhibiting the level or activity of a protein encoded by SEQ ID NO: 1, preferably, SEQ ID NO: 2 and/or SEQ ID NO: 3 or by a homologous human gene can be used. The inhibitor may be a siRNA or shRNA specific to a SEQ ID NO: 1, preferably, SEQ ID NO: 2, SEQ ID NO: 3 and/or a homologous human gene thereof.

The amino acid sequence of the protein encoded by the sequence set forth in SEQ ID NO: 1 is SEQ ID NO: 4, the amino acid sequence of the protein encoded by the sequence set in SEQ ID NO: 2 is SEQ ID NO: 5, and the amino acid sequence of the protein encoded by the sequence in SEQ ID NO: 3 is SEQ ID NO: 6.

In another aspect, an activator of the level or activity of a protein which is normally activated or upregulated by caspase-8 activity in the skin, can be used in the treatment or prevention of an inflammatory skin disease, disorder or condition. These proteins can be identified or screened by methods according to the invention to identify or screen of a target protein involved in the pathology or course of a skin inflammatory disease disorder or condition as described below.

Caspase -8 may be used in a variety of ways and routes. For example, the agent or molecule can be used topically, into, to, or on the skin or can be adsorbed through epithelial or endothelial tissues.

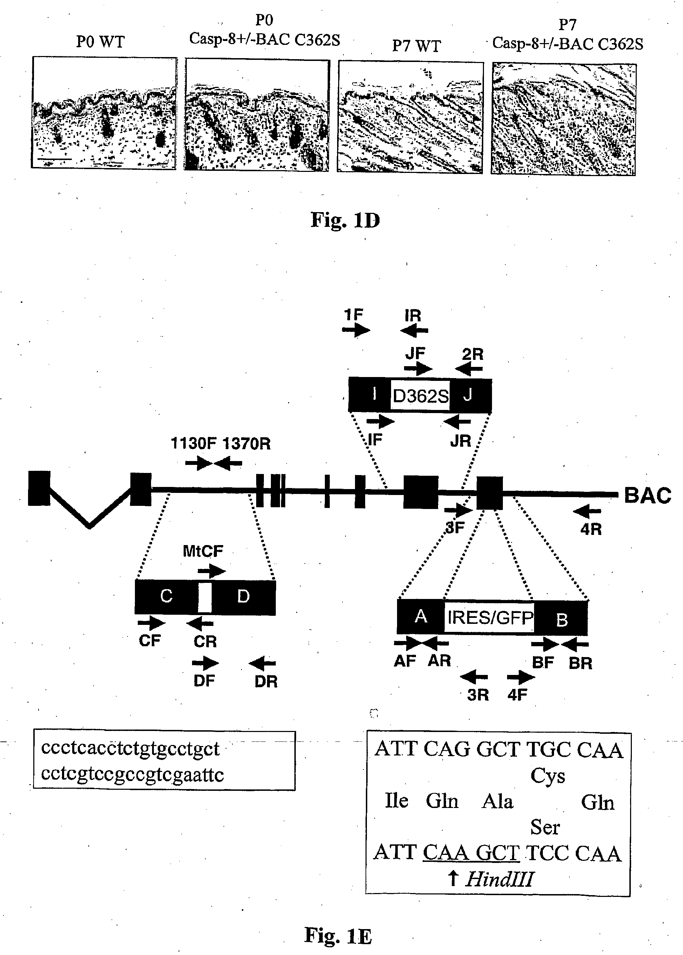

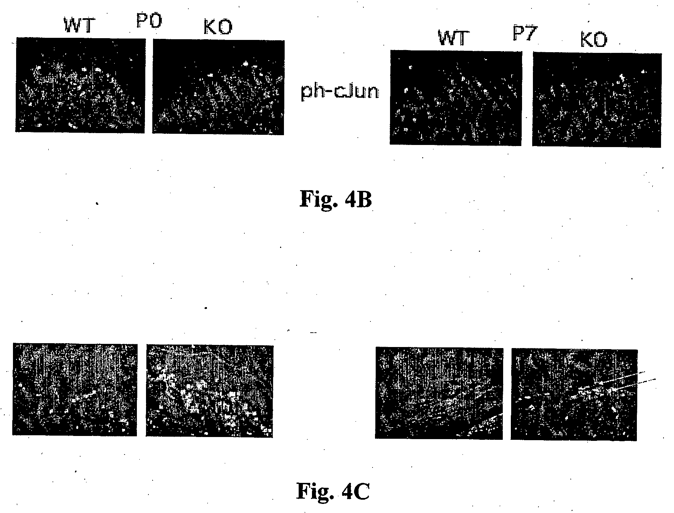

We found according to the invention, that in spite that the inflammatory hyperplasia mediated by caspase-8 deficiency displayed enhanced cell division in the basal epithelial layer and withheld differentiation, the pathological state was not associated with deficient apoptosis. In fact, the incidence of dying cells in the Casp-8^{-/+}/BAC-C362S and Casp-8^{f1/-}K5-Cre epithelium was higher than in normal epithelium **(****Fig. 4C****).** In addition, the pathological state in the caspase-8-defficient epithelium was not associated with arrest of keratinocyte death and cornification failure **(****Fig. 1D****).** Indeed, although cornification results from keratinocyte death, this death does not manifest most of the characteristic features of apoptosis.

Only one caspase, caspase-14, of yet unknown function, is uniquely expressed in epithelial cells, and has reproducibly been shown to be cleaved during the cornification (Takahashi et al., 1998, Lippens et al., and Lippens et al., 2003). We have found according to the invention, that cleavage or activation of caspase-14 is not affected in the inflammatory hyperplasia mediated by caspase-8 deficiency.

It has been speculated in the art that the nonapoptotic functions of caspase-8 do not depend on its enzymatic activity, since overexpression of enzymatically inactive caspase-8 can trigger NF-κB activation, and in view of the evidence for signaling activities of cFLIP, an enzymatically inactive caspase-8 homologue that seems to participate in at least part of the caspase-8 nonapoptotic functions. However, the contribution of caspase-8 to T cell growth has recently been shown in the art to depend on its enzymatic function. In the latter case, evidence was presented for a role of the caspase-8 enzymatic function in activation of the p65/p50 NF-κB dimers. We have found according to the invention that the role of caspase-8 in epidermal differentiation does depend on its enzymatic proficiency.

Although the pathology of the caspase-8 deficient epidermis somewhat resembles that observed in the skin lacking p65 NF-κB (Zhang et al, 2004), the cellular events affected in these two cases seem to differ. For example, arrest of p65 NF-κB activation in epidermis results in excessive growth of the keratinocytes, which unlike the case of capsase-8 deficiency, is manifested by a marked thickening of the basal layer and seems not to affect keratinocyte differentiation. Moreover, while the skin pathology consequent from the arrest of NF-κB activation in the keratinocytes results from excessive increase in Jun phosphorylation, and fully depends on TNF function, the pathology resulting from caspase-8 deficiency seems not to involve any increase in Jun phosphorylation and, even though enhanced by TNF, it does not fully depend on it. Thus, it seems unlikely that the pathology resulting from caspase-8 deficiency reflects a role of caspase-8 in regulation of activation of the p65/p50 NF-κB dimers. In fact, we found that there is no difference between the extents of nuclear translocation of these NF-κB proteins in the model mice of caspase-8 deficient epithelium compared to normal mice.

Our findings show that mice with a keratinocyte-specific deletion of the caspase-8 gene developed an inflammatory skin disorder that was, in part, perpetuated by TNF. However, we found according with the invention that the differentiation insufficiency that initiates the pathology reflects a cell-autonomous role of caspase-8, which is independent of TNF, yet depends on the caspase-8 enzymatic activity.

The results obtained according to the invention show that a skin disease, that may be optimally treated or prevented by administering an agent of the invention is a skin disease exhibiting one or more histological and/or immunological features resembling those found in the inflammatory disease that is mediated by caspase-8 deficiency in the skin and particularly in the epidermis, said skin disease may comprise one, more than one, two, three, four, five, six, seven, eight or all of the following features: (i) accumulation of leukocytes in the dermis, mainly mononuclear phagocytes and/or eosinophils; (ii) lack of increase of either T or B lymphocytes; (iii) expression of Keratin 6 in the epidermis specifically at the site of the epithelial lesion; (iv) significant suprabasal expression of keratin 14 (K14); (v) K6 and K14 expression in all viable layers; (vi) reduced or absent expression of the suprabasal keratin 1 (K1); (vii) reduced or absent expression of one or more skin differentiation markers selected from keratin 1, loricrin and filaggrin; (viii) increased expression of one, more than one, two, three, four , five, six, seven, eight, nine, or all of the following proteins: ISG15 (G1p2), Cxc110, 9230117N10Rik (IL33)-human orthologue is called C90rf26, IL-19, Sprr2f, S100a9, IL-6, MMP 13, Ccl3, IL1b; (ix) increased expression of a human protein homologous to the one encoded by SEQ ID NO:2 or by SEQ ID NO: 3; (x) increase of TH2 response, as supported by elevated expression of IL-4, IL-5 (evidenced by the increase in eosinophiles that is mediated by this cytokine, Kupper et al., 2004) and IL-19; (xi) activation of Stat-1 and Stat-3 in the epidermis; (xii) upregulation of S100A8 in the dermis; (xiii) and no impairment of caspase-14 processing. For example, an agent according to the invention can be used to treat a disease like AD since AD has features resembling that found in the epidermis of the Casp-8^{f1/-}K5-Cre mice and in the Casp-8-/+/BAC-C362S mice such as accumulation eosinophils and increase of TH2 response.

Thus, a disease that may be optimally treated or prevented by administering an agent selected from, caspase-8 or a fragment thereof includes, but is not limited to, psoriasis and atopic dermatitis.

Further aspects of the invention relate to methods for identifying target proteins involved in the pathology or course of an inflammatory skin disease, disorder or condition. According to the present invention, we have developed a method for identifying a target protein involved in the pathology or course of a skin disease, disorder or condition whose level of expression or activity is normally regulated by caspase-8 activity in the skin comprising the steps of: comparing the profile of gene expression in a sample of skin comprising epithelial keratinocytes arrested in caspase-8 expression level or activity to the profile of gene expression in a sample of skin comprising epithelial keratinocytes having normal caspase-8 expression level or activity; assessing a gene whose expression is normally upregulated or downregulated by caspase-8 in the sample of skin comprising epithelial keratinocytes having normal caspase-8 expression level or activity, and whose expression is downregulated or upregulated in the sample comprising epithelial keratinocytes arrested in caspase-8 expression level or activity, respectively, wherein a gene whose expression is downregulated or upregulated in the sample comprising epithelial keratinocytes arrested in caspase-8 expression level or activity encodes a candidate target protein involved in the pathology or course of said skin disease, disorder or condition.

In certain embodiments, the method of the invention employs a sample of skin that is from a human or mouse origin.

The profile of expression of a gene can be measured by tools that are well known in the art such as microarray analysis, Western blotting analysis, in situ hybridization, and RT-PCR, as shown in the Examples below. The profile of gene expression is monitored by gene arrays, for example as carried out in the non-limiting examples.

By using the method of the invention we identified the following genes (and encoded proteins) whose expression is upregulated in samples of skin comprising epithelial keratinocytes arrested in caspase-8 activation; ISG15 (G1p2), Cxcl10, 9230117N10Rik (IL33)-human orthologue is called C90rf26, IL-19, Sprr2f, S100a9, IL-6, MMP13, Ccl3, IL1b. These genes are involved in inflammatory processes. Thus, in one embodiment of the invention, the method allows the identification of a target gene (or the encoded protein) involved in the pathogenesis or course of a skin inflammatory disease, disorder or condition such as psoriasis and -atopic dermatitis. Proteins encoded by SEQ ID NO: 1, and proteins of unknown function encoded by 9230117E20Rik (SEQ ID NO: 2) and 2010002N04Rik (SEQ ID NO: 3) have been positively selected herein by the method of the invention. Since these proteins or homologous human proteins are regulated by caspase-8 in the skin, they play a role in development and/or homeostasis of the skin and therefore are candidates to be target proteins involved in the pathology or course of an inflammatory skin disease, disorder or condition.

Thus, an inhibitor of expression of a gene corresponding to SEQ ID NO: 1, SEQ ID NO: 2, and/or SEQ ID NO: 3, or a homologous human gene thereof and/or an inhibitor of a protein encoded by said gene may be beneficial for the treatment or prevention of a skin inflammatory disease, disorder or condition such as atopic dermatitis or psoriasis, and can be used in the manufacture of a medicament for the treatment or prevention of an inflammatory skin disease disorder or condition.

Pharmaceutical compositions comprising a pharmaceutically acceptable carrier and an inhibitor of a gene comprising the sequence selected from SEQ ID NO: 1, SEQ ED NO: 2, and SEQ ID NO: 3, or of a homologous human gene thereof and/or an inhibitor of a protein encoded by said gene, for example may be used for the treatment or prevention of a skin inflammatory disease, disorder or condition such as atopic dermatitis or psoriasis.

The inhibitor in the pharmaceutical composition may be a siRNA or shRNA specific to a SEQ ID NO: 1, preferably, SEQ ID NO: 2, SEQ ID NO: 3 or of a homologous human gene thereof.

Publications relating to the function of caspase-3 (Okuyama et al., 2004), a major effector caspase, revealed that apart from its participation in death induction by a variety of agents, caspase-3 is involved, at a particular phase of during embryonic development known as midgestation, in the induction of differentiation of the skin epidermis in mouse. In fact, the enzymatic activity of caspase-3 is essential for maintaining the keratinocyte commitment to terminal differentiation during embryonic development of the skin.

We found according to the invention that although the Casp8BAC-C362S transgenic is expressed in the mouse, just as the endogenous caspase-8, at early stages of embryogenesis, and the keratin-5-induced expression of Cre in Casp-8^{f1/-} K5-Cre mice occurs in embryogenesis stages, the skin pathology in these mice was not apparent earlier than three days after birth. At the time of birth, the histological features of the skin were indistinguishable from that of the wild-type mice, suggesting that, unlike caspase-3, caspase-8 is dispensable for embryonic development of the skin epithelium.

It has been shown according to the invention that caspase-8 plays a similar role to caspase-3 in the postnatal epidermal morphogenesis since targeted elimination of caspase-8 expression in the skin resulted in increased proliferation and decreased expression of the late differentiation markers in the postnatal, but not in the embryonic keratinocytes.

The contribution of caspase-3 to the regulation of keratinocyte differentiation at a distinct phase of midgestation has been correlated to a Notch1-induced increase in expression of caspase-3 in this phase, and was suggested to reflect a role of caspase-3-induced processing of PKC-d in activation of the latter kinase. In contrast, we found that caspase-8 deficiency compromises the downregulation of p21, which precedes the induction of the differentiation proteins in the keratinocytes.

Normal homeostasis of the skin and pathological aberrations of the skin are affected by a constant cross-talk between the epidermis and the dermis, as well as among the cells of each of these two layers, through the release of various soluble mediators. We found according to the present invention that the epidermal pathology observed in the absence of caspase-8 is associated with accumulation of inflammatory cells, mainly leukocytes, in the dermis and is strongly affected by the function of TNF, produced by these inflammatory cells, by the keratinocytes themselves, or perhaps by both. Through assessment of the differentiation capacity of the keratinocytes in culture we found that the differentiation program of these cells shifts to a caspase-8 dependent state at about one day after birth, clearly before the accumulation of leukocytes in the dermis, and that this shift occurs independently of TNF. Several differentiation-related keratinocyte proteins were found to be affected by caspase-8 deficiency at about one day after birth. However, pathological manifestations in vivo, appear only later, at a rate that does depend on TNF, and perhaps on some other leukocytes-produced mediators. Thus, our results show that TNF function and the accumulation of leukocytes are not central to the change that occurs in the epithelium but just serve auxiliary roles in it.

Another aspect of the invention relates to methods for screening a candidate compound for treating a skin inflammatory disease, disorder or condition. The above findings with cultures of isolated keratinocytes deficient in caspase-8 showing that differentiation-related keratinocyte proteins are affected by caspase-8, paved the way to the development of a method for screening of a candidate compound for treating a skin inflammatory disease, disorder or condition. The method according to the invention comprises, providing a culture of cells comprising keratinocytes arrested in caspase-8 expression level or activity, introducing a test compound in said cells, inducing differentiation of the cells by increasing the calcium concentration in the culture medium of the cells, measuring in the cells the expression level of a differentiation marker of the skin and/or p21ARF in the presence or absence of the test compound, wherein increase in the expression level of a differentiation marker of the skin and/or decrease of p21ARF in the presence of the test compound is indicative that the test compound is a candidate compound for treating or preventing said disease, disorder or condition. Culture of cells comprising keratinocytes arrested in caspase-8 expression level or activity can be isolated from mice expressing transgenic inactive mutant caspase-8 or mice exhibiting conditional caspase-8 knockout in the skin, for example, as shown in the examples. A candidate compound may be screened from libraries of chemicals or natural agents. Introducing the test compound in the cells may be carried out for example by adding the test compound in the culture medium of the cells. Measuring the expression level of the proteins in the cells can be done by RT-PCR analysis, or by immunoassay analysis as carried out in the examples below employing protein specific antibody. In one embodiment of the invention, the method measures the levels of keratin 1, filagrin and/or loricrin, for example by immunoassay analysis.

According to the present invention, we have generated an animal model of an inflammatory skin disease, disorder or condition by developing an animal arrested in keratinocyte caspase-8 expression level or activity. The arrest of caspase-8 activity or level can be achieved as shown in the non limiting examples by knocking out caspase-8 in epithelial keratinocyte of the animal or by developing animals having endogenous caspase-8 but expressing also transgenic caspase-8 lacking enzymatic activity such as BAC-C362S in which the Cys at residue 362 has been replaced for Ser. In one embodiment of the invention the animal is mouse.

According to the invention, this animal model can be used to test the efficacy of a candidate compound for treating a skin inflammatory disease, disorder or condition such as atopic dermatitis or psoriasis. Thus in one aspect, the invention relates to a method to test the efficacy of a candidate compound for treating a skin inflammatory disease, disorder or condition comprising administering to an animal model generated according the invention a candidate test compound and assessing prevention or reduction of skin pathology in said animal model.

The results obtained by RT-PCR analysis confirmed that genes ISG15 (G1p2), Cxc110, 9230117N10Rik (IL33)-human orthologue is called C90rf26, IL-19, Sprr2f, S100a9, IL-6, MMP13, Ccl3, IL1b, the human homologous gene of 9230117E20Rik (SEQ ID NO: 2), the human homologous gene of 2010002N04Rik (SEQ ID NO: 3) are unpregulated compared to the levels of expression of said genes in a sample of skin from a normal subject and that the same pattern of upregulation can be detected before the pathology develops in the skin (Example 6).

Thus, another aspect of the invention relates to methods for diagnosing an inflammatory skin disease, disorder or condition or a predisposition to develop said disease disorder or condition in an individual. In view of the findings of the present invention, the levels of caspase-8 activity or expression and/or expression of genes that are normally regulated by caspase-8 in the skin can be examined in a sample of skin or epidermis of an individual in order to find out whether the individual suffers or is likely to suffer of an inflammatory skin disease, disorder or condition. For example, if in a skin sample of a tested individual the levels of caspase-8 activity or expression are downregulated and/or the levels of expression or activity of one, more than one, two, three, four, five, six, seven, eight, nine, or all of the following genes (or encoded proteins) including, but not limited to, ISG15 (G1p2), Cxc110, 9230117N10Rik (IL33)-human orthologue is called C90rf26, IL-19, Sprr2f, S100a9, IL-6, MMP13, Ccl3, IL1b and at least one gene of unknown function such as the human homologous genes of 9230117E20Rik (SEQ ID NO: 2) and 2010002N04Rik (SEQ ID NO: 3) are unpregulated, for example, by at least 15%, or up to 17%, 25%, 33%, 50%, 67%, and/or 100% compared to the levels of expression of said genes in a sample of skin of a healthy individual, one can assume that the tested individual has an inflammatory skin disease, disorder or condition or is likely to have or develop said skin disease, disorder or condition.

Thus in another aspect, the invention relates to a method for diagnosing in a tested individual a skin disease, disorder or condition associated with caspase-8 deficiency in epithelial keratinocytes or a predisposition or likelihood to develop said skin disease, disorder or condition, comprising measuring in a sample of skin of the tested individual and in a sample of skin of at least one healthy control individual the caspase-8 level or activity, and/or expression of genes that are normally regulated by caspase-8 in the skin and are unregulated in the skin disease, disorder or condition, and/or detecting aberrations in nucleic acid encoding caspase-8, wherein a decrease of the levels of caspase-8 activity or level, and/or presence of aberrations in nucleic acid encoding caspase-8, and/or unregulation of expression of genes that are normally regulated by caspase-8 in the skin in the tested individual compared to level, activity or expression in the skin of the said at least one healthy control individual is indicative of said skin disease, disorder or condition or of a likelihood or predisposition to develop said skin disease, disorder or condition in the tested individual.

Detecting nucleic acid aberration of caspase-8 in a sample of a tested individual can be carried out by methods well known in the art such as isolating RNA from a sample of skin of the tested individual, for example, as described in the examples below and sequencing caspase-8 of PCR amplified cDNA.

Characteristic activity of caspase-8 is its proteolytic activity at specific substrate sites. Thus, activity of caspase-8 in a sample can be determined by means of routine experimentation comprising subjecting such sample e.g. to a substrate as described in example 3 of US Patent 6,399,327.

Measuring the expression level of caspase-8 in the skin can be carried out by methods well known in the art such as by subjecting the sample to immunoassay employing caspase-8 specific antibody or by extracting RNA from the sample and employing RT-PCR using caspase-8 specific primers. Typically, RT PCR analysis include RT-PCR of a house keeping gene such as beta actin, as shown in the examples below, as control for sample load.

Thus, the invention provides a method for diagnosing in an individual a skin disease, disorder or condition associated with caspase-8 deficiency in the skin or the predisposition to develop said skin disease, disorder or condition, comprising, measuring in a sample of skin of the tested individual and in a sample of skin of at least one healthy control individual the expression level or activity of caspase-8 and/or detecting aberrations in nucleic acid encoding caspase-8 in a sample of skin from the tested individual, wherein detection of a decrease of caspase-8 activity or expression level in the sample of the tested individual as compared to the sample of said at least one healthy control individual, and/or detection of aberrations in nucleic acid encoding caspase-8 in the tested individual is indicative of said skin disease, disorder or condition, or of a predisposition to develop said skin disease, disorder or condition in the tested individual.

The expression level or activity of a protein encoded by a human gene homologous to a gene set forth in SEQ ID NO: 1, SEQ ID NO: 2, and/or SEQ ID NO: 3, wherein detection of an increase of the expression level or activity of a protein encoded by a human gene homologous to the gene set forth in SEQ ID NO: 1, SEQ ID NO: 2, and/or SEQ ID NO: 3 in the sample of the tested individual as compared to the sample of said at least one healthy control individual could be indicative of a skin disease, disorder or condition or of a predisposition to develop said skin disease, disorder or condition in the tested individual.

The expression level of a protein can be measured as exemplified below by methods well known in the art such as immunoassay and RT PCR.

In a further embodiment, the method is for diagnosing atopic dermatitis or for diagnosing predisposition to develop the disease.

Diagnosing in an individual an inflammatory skin disease, disorder or condition or a predisposition to develop said skin disease, disorder or condition, may comprise measuring in a sample of skin of the tested individual and in a sample of skin of at least one healthy control individual the expression level or activity of one, more than one, two, three, four, five, six, seven, eight, nine, or all of the following proteins selected from ISG15 (G1p2), Cxc110, 9230117N10Rik (IL33)-human orthologue called C90rf26, IL-19, Sprr2f, S100a9, IL-6, MMP 13, Ccl3, or IL1b, and of a protein encoded by a human gene homologous to 9230117E20Rik (SEQ ID NO: 2), or a protein encoded by a human gene homologous to 2010002N04Rik (SEQ ID NO: 3), wherein detection of an increase of the expression level or activity of said one, more than one, two, three, four, five, six, seven, eight, nine, or all of the following proteins selected from ISG15 (G1p2), Cxc110, 9230117N10Rik (IL33)-human orthologue called C90rf26, IL-19, Sprr2f, S100a9, IL-6, MMP13, Ccl3, or IL1b, and of a protein encoded by a human gene homologous to 9230117E20Rik (SEQ ID NO: 2), or a protein encoded by a human gene homologous to 2010002N04Rik (SEQ ID NO: 3)in the sample of the tested individual as compared to the sample of said at least one healthy control individual is indicative of said skin disease, disorder or condition or of a predisposition to develop said skin disease, disorder or condition in the tested individual.

In certain embodiments, the methods of the invention employ the sample of at least one a healthy control individual, in other embodiment of the invention a sample of a single healthy control individual, in further embodiments of the invention individual samples of more than one, two or three healthy control individuals or a pool of samples of two three or more healthy control individuals.

As used herein, a "gene" refers to a polynucleotide or portion of a polynucleotide comprising a sequence that encodes a protein. It is well understood in the art that a gene may also comprise non-coding sequences, such as 5' and 3' flanking sequences (such as promoters, enhancers, repressors, and other regulatory sequences) as well as introns.

The term "homologous" or "homologue" or "ortholog" is known and well understood in the art and refers to related sequences that share a common ancestor and is determined based on degree of sequence identity. These terms describe the relationship between a gene found in one species and the corresponding or equivalent gene in another species. For purposes of this invention homologous sequences are compared. "Homologous sequences" or "homologues" or "orthologs" are thought, believed, or known to be functionally related. A functional relationship may be indicated in any one of a number of ways, including, but not limited to, (a) degree of sequence identity (b) same or similar biological function. Preferably, both (a) and (b) are indicated. The degree of sequence identity may vary, but is preferably at least 50% (when using standard sequence alignment programs known in the art), more preferably at least 60%, more preferably at least about 75%, more preferably at least about 85%. Homology can be determined using software programs readily available in the art, such as those discussed in Current Protocols in Molecular Biology (F. M. Ausubel et al., eds., 1987) Supplement 30, section 7.718, Table 7.71. Preferred alignment programs are MacVector (Oxford Molecular Ltd, Oxford, U.K.) and ALIGN Plus (Scientific and Educational Software, Pennsylvania). Another preferred alignment program is Sequencher (Gene Codes, Ann Arbor, Mich.), using default parameters.

The terms "polypeptide," "peptide," and "protein" are used interchangeably herein to refer to polymers of amino acids of any length. These terms also include proteins that are post-translationally modified through reactions that include glycosylation, acetylation and phosphorylation.

The disclosure provides also, a method of treatment of a skin inflammatory disease, disorder or condition comprising administering to a subject in need a therapeutically effective amount of a molecule or an agent selected from: caspase-8 or a fragment thereof; a polynucleotide encoding caspase-8 or a fragment thereof; a vector comprising said polynucleotide; an activator of the level or activity of caspase-8; an inhibitor of a natural inhibitor of casapase-8 activation; an inhibitor of the level or activity of a protein which is normally downregulated by caspase-8 activity in the skin; and an activator of the level or activity of protein which is normally upregulated by caspase-8 in the skin.

The agent or molecule according to the disclosure can be administered to an individual in a variety of ways. In one embodiment, the agent or molecule is administered topically, into, to, or on the skin. Any other therapeutically efficacious route of administration can be used, for example absorption through epithelial or endothelial tissues or by gene therapy wherein a DNA molecule or polynucleotide encoding the agent is administered to the patient (e.g. via a vector), which causes the active agent to be expressed and secreted in vivo. In addition active ingredients according to the disclosure can be administered together with other components of biologically active agents such as pharmaceutically acceptable surfactants, excipients, carriers, diluents and vehicles.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition can be administered to an individual in a variety of ways or routes. The active ingredients are administered topically, into, to or on the skin. Any other therapeutically efficacious route of administration can be used. Active ingredients may be absorbed through epithelial or endothelial tissues. The active ingredient may be administered by gene therapy wherein a DNA molecule encoding the active agent is administered to the patient (e.g. via an expression vector), which causes the active agent to be expressed and secreted in vivo. In addition active ingredients according to the invention can be administered together with other components of biologically active agents such as pharmaceutically acceptable surfactants, excipients, carriers, diluents and vehicles.

Other routes of administration include intraliver, intradermal, transdermal (e.g. in slow release formulations), intramuscular, intraperitoneal, intravenous, subcutaneous, oral, epidural, topical, and intranasal routes. In addition the substance can be administered together with other components of biologically active agents such as pharmaceutically acceptable surfactants, excipients, carriers, diluents and vehicles.

The active ingredients can be formulated as a solution, suspension, emulsion or lyophilized powder in association with a pharmaceutically acceptable vehicle (e.g. water, saline, dextrose solution) and additives that maintain isotonicity (e.g. mannitol) or chemical stability (e.g. preservatives and buffers). The formulation is sterilized by commonly used techniques.

A "therapeutically effective amount" is such that when administered, the active ingredient results in prevention of the pathology, amelioration or beneficial improvement in the course of the disease. The dosage administered, as single or multiple doses, to an individual will vary depending upon a variety of factors, including pharmacokinetic properties of active ingredients, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining prevention of the pathology, amelioration or beneficial improvement in the course of the disease in an individual.

### EXAMPLES

### MATERIALS AND METHODS

**(i) *Modifcation of a Bacterial artificial chromosome (BAC) comprising caspase-8.*** A BAC clone from the RPCI-24 mouse (C57BL/6) BAC library, encompassing caspase-8, (RP24-238B22) was obtained from CHORI (BACPAC Resources). DH10B bacteria harboring the BAC clone were grown in LB medium containing 12.5mg/ml of chloramphenicol. The presence of caspase-8 in the bacterial colonies was verified by PCR using oligonucleotides Exon8F-8R, Exon1F-1R, 5'UTRF-R [the oligonucleotides used are listed in section (i) below]. Modification of the BAC clone was performed essentially as described (Gong et al., 2002 and Sparwasser et al, 2004), using the pDelsac shuttle vector both for deleting the SacB gene from the RP24-238B22 clone and for modification of the caspase-8 gene. The following four modifications were introduced (see schematic Fig. 1E):
a. To monitor the expression of the transgenic in vivo, we placed an IRES sequence and the GFP cDNA, flanked by two sequences for the FRT recombination site, downstream of the stop codon of caspase-8. To introduce this sequence into the shuttle vector, the homology arms upstream and downstream of the stop codon ('Box A' and 'Box B', see diagrammatic presentation of the targeting cassettes in **Fig. 1E****),** cloned from the RP24-238B22 DNA by PCR using olignucleotides AF-AR, AF-AR2 and BF-BR respectively, were introduced to the Sa1I-EcoRI and SpeI-NotI sites of the pBC FRT-IRES/GFP-FRT vector. The composite insert was then transferred to the shuttle vector.
b. To further assist monitoring the expression of the transgenic, we fused the T7 tag to the C-terminus of caspase-8 by introducing the T7 coding sequence to the EcoRI site in pBC FRT-IRES/GFP-FRT containing BoxA (derived from AF-AR2 PCR product) and BoxB of 'a' above.
c. To ease genotyping the transgenic mice, we exchanged a 20 nucleotide sequence within the first caspase-8 intron with a unique sequence (with equal nucleotide proportions). BoxC and BoxD were amplified using primer CF-CR and DF-DR. These two PCR products were used as a template in second PCR together with primer CF and DR that resulted in the fusion of two PCR fragments.
The findings presented in this study concern mice that express the BAC with all the above three modifications. Identical findings were reached with mice expressing BAC to which only the first modification (IRES/GFP) was introduced.
d. A catalytically inactive caspase-8 transgenic was generated by replacing the active-site cysteine at position 362 with serine (C362S), BoxI and BoxJ that border this sequence were amplified and fused by PCR using primers Box1S-IR and JF-Box2AS
All the above modification-cassettes were cloned into AscI-NotI sites of the pDelsac shuttle vector.
Following electroporation of the shuttle constructs to BAC-containing bacteria, and selection with chloramphenicol and ampicillin, colonies that had integrated the shuttle vector were identified by direct PCR or restriction enzyme analysis using the following primer pairs: TB3F-TB3R for AB or ATBCD, MutCF-1370R for CD, IF2-IR1and HindIII for IJ).
Two or three positive colonies of each kind were then cultured in 5% sucrose to select for resolved BACs, followed by further selection by picking ampicilin-sensitive colonies. Positive colonies were validated by PCR (TB3F-TB3R, TB4F-TB4R for AB or ATBCD, 1130F-1370R, MutCF-1370R for CD, IF2-JR1 and HindIII for IJ) and restriction enzyme analysis as described above.

**oligonucleotides used:**

| Primer | Sequences | Position |
|---|---|---|
| SEQ ID NO: 7 | | |
| Exon8F | TTAGCATCCTGACTGGCGTGA | Exon8 |
| SEQ ID NO: 8 | | |
| Exon8R | AAGCCATGTGAACTGTGGAGAGC | Exon8 |
| (SEQ ID NO: 9 | | |
| Exon1F | GAAGACCTGGCTGCCCTCAA | Exon1 |
| SEQ ID NO:10 | | |
| Exon1R | GGATCCCGCAGCTCTCTCAC | Exon1 |
| SEQ ID NO: 11 | | |
| 5'UTRF | CTCTAGGGCTGGCACCAGGA | 5'UTR |
| SEQ ID NO: 12 | | |
| 5'UTRR | CCGGCTCACAGAGGTTTGCT | 5'UTR |
| SEQ ID NO: 13 | | |
| BoxCF | GGCGCGCCtacatacgcctaggaaggaccctgt | Intron1 |
| SEQ ID NO: 14 | | |
| BoxCR | gaattcgacggcggacgaggaggtgtctgcctatgactctgttgcttgcctttggattcc | Intron1 |
| SEQ ID NO: 15 | | |
| BoxDF | cctcgtccgccgtcgaattcccagttattggagaacccacatgaagaccaagctgcacat | Intron1 |
| SEQ ID NO: 16 | | |
| BoxDR | AAGGAAAAAAGCGGCCGCgttactcaggttacgtgggggaaag | Intron 1 |
| SEQ ID NO: 17 | | |
| BoxAF | | Exon8 |
| SEQ ID NO: 18 | | |
| BoxAR | GGAATTCTTAGGGAGGGAAGAAGAGCTTCTTCCG | Exon8 |
| SEQ ID NO: 19 | | |
| BoxAR2 | GGAATTCGGGAGGGAAGAAGAGCTTCTTCCG | Exon8 |
| SEQ ID NO: 20 | | |
| BoxBF | GACTAGTTGATGTGTGCTCTCCACAGTTCAC | 3'UTR |
| SEQ ID NO: 21 | | |
| BoxBR | | |
| SEQ ID NO: 22 | | |
| Mut CF | CCT CGT CCG CCG TCG AAT TC | Intron 1 |
| SEQ ID NO: 23 | | |
| IN1-1130F | CCC TCA CCT CTG TGC CTG CT | Intron 1 |
| SEQ ID NO: 24 | | |
| IN1-1370R | gctcggggagtcttgtggaa | Intron 1 |
| SEQ ID NO: 25 | | |
| BoxIF2 | aggcgcgcctgcctacaggctgtgttctg | |
| SEQ ID NO: 26 | | |
| BoxJR1 | aaggaaaaaagcggccgcctctaaccacagaaatgagtaaggaagcat | |
| SEQ ID NO: 27 | | |
| TB3F | tggtttcttgtaaccagcagag | In BoxA |
| SEQ ID NO: 28 | | |
| TB3R | agacccctaggaatgctcgt | In IRES |
| SEQ ID NO: 29 | | |
| TB4F | acatggtcctgctggagttc | In GFP |
| (SEQ ID NO: 30 | | |
| TB4R | attcaccccattctgctgac | In BoxB |
| SEQ ID NO: 31 | | |
| Box1S | ttggcgcgcctctatgcaagagcagcaaggactct | |
| SEQ ID NO: 32 | | |
| Box2AS | ttgcggccgcgctgttctggcaactcagttttctc | |
| SEQ ID NO: 33 | | |
| BoxIR | cctttctggaagttacttccttgggaagcttgaatg | |
| SEQ ID NO: 34 | | |
| BoxJF | cattcaagcttcccaaggaagtaacttccagaaagg | |

***(ii) Isolation of BAC DNA for microinjection.*** BAC DNA was isolated by double acetate precipitation and cesium chloride gradient ultracentrifugation. After wash with ethanol, the BAC DNA was dissolved in TE buffer, lanearized by PI-SceI endonuclease (NEB) and drop-dialyzed for 6 hrs against microinjection buffer (10 mM Tris, pH7.5, 0.1 mM EDTA, pH8.0 and 100 mM NaCl) by floating on 0.025 µm Millipore membrane filter disc. The quality and quantity of BAC DNA was assessed by pulse-field gel electrophoresis (PFEG; 5 V/cm, 120 angle, lanear ramping time of 5-120 s for 24-30 hrs) in 1% agarose using CHEF-DR III PFEG system (Bio-Rad, check). The DNA was diluted to 2ng/ul in injection buffer (10 mM Tris, pH7.5, 0.1 mM EDTA, pH8.0 and 100 mM NaCl) and mixed with equal volume of 2x polyamine (60 mM spermine and 140 mM spermidine in injection buffer) for the injection.
***(iii) Generation and analysis of BAC-transgenic mouse.*** The DNA was injected at concentration of 1ng/µl into the pronucleus of fertilized oocytes derived from CBF1 or C57BL/6 mice. Transgenic mice were identified both by PCR analysis (using primers Mut CF and IN1-1730R) of genomic DNA prepared from tail biopsies and by FACS analysis for GFP expression in the peripheral-blood leukocytes.
The catalytically inactive caspase-8 transgenic was distinguished from the active one by using primers IF2-JR1 for PCR followed by digestion with HindIII.
For Western blot analysis of various tissue extract from BAC tg mouse, tissue lysates from wild type and BAC transgenic mice were prepared by homogenization in lysis buffer (1% SDS, 1mM sodium orthovanadate, 10mM Tris pH 7.4) following by boiling for 5min. Protein concentration was determined with the BCA protein assay kit (Pierce). Aliquots of 25µg protein were then analyzed by SDS-PAGE followed by immunoblotting with anti-mouse caspase-8 (3B10, kindly provided by Dr. A Strasser), anti-GFP and anti-human b-actin monoclonal antibodies (Sigma).
**(iv)** ***Development of K5 Cre** c****asp8*^{*fl*/+} *and K5 Cre casp8*^{*fl*/-}*mice.*** Mice in which one of the caspase-8 alleles was knocked out (casp-8^{+/-}, Varfolomeev et al., 1998) were mated with a transgenic mouse lane that expressed Cre under control of the basal epithelia specific Keratin-5 promoter (K5 Cre Casp-8+, Ramirez et al., 1994). These K5 Cre Casp-8+mice were then mated with mice carrying homozygous conditional caspase-8 allele (Casp-8flfl , Kang et al., 2004) to yield K5 Cre casp8^{fl/+} and K5 Cre casp8^{fl/-}mice.
***(v) Histology and immunostaining.*** Skin and other organs were fixed in 10% phosphate-buffered formalin pH 7.4, embedded in paraffin, cut into 4-µm sections, and stained with hematoxylin and eosin. Adjacent sections were used for immunostaining with the indicated antibodies. Cy-3 coupled secondary antibodies (Jackson Immunoresearch Labs) were used. Sections were further counterstained with Hoechst 33342 (Sigma) to visualize nuclei.
TUNEL staining kit (Roche Diagnostics) was used for detection of apoptotic cells.
Rabbit anti-cytokeratin 1, 6, 14, involucrin, filaggrin and loricrin antibodies were purchased from Covance. Mouse anti-p21 was purchased from Pharmingen. Rat anti-F4/80 was purchased from Serotec. Rat anti-Ki67 was purchased from DAKO.
*(**vi**) **Preparation of a keratinocyte culture.*** Primary keratinocytes were isolated and cultured from newborn to four-days-old mice. Epidermis was separated from dermis with 3.3% tyrpsin (Gibco) overnight at 4°C. Keratinocytes were dissociated by shaking for 15min in MEM medium and plated onto dishes with MEM medium supplemented with 10% chelex-treated fetal calf serum, antibiotics, KCl (400µg) and 3% sodium bicarbonate.

### Example 1: Normal growth and differentiation of the epidermal keratinocytes depend on caspase-8 function.

To explore structure-function relationship in caspase-8 for the various effects that this enzyme exerts in vivo, we employed bacterial artificial chromosome (BAC) mediated transgenics (for details see Materials and Methods) to generate mice that apart from their endogenous caspase-8 alleles express an additional copy of the caspase-8 gene, or various mutants thereof **(****Fig. 1A****).** These transgenics were expressed ubiquitously, manifesting the same tissue-specific expression pattern as that observed for the endogenous gene **(****Fig. 1B****).** Mice expressing the wild type transgenic (Casp-8+/+/BAC-WT) appeared normal, even after deletion of the endogenous caspase-8 alleles by mating these mice with caspase-8 knockout mice. On the other hand, mice expressing an active-site mutant of the enzyme, devoid of enzymatic activity (Casp-8BAC-C362S) developed skin pathology **(****Fig. 1C****).** Mice that in addition to this transgenic mutant caspase-8 also possessed the two endogenous wild-type caspase-8 alleles (Casp-8+/+/BAC-C362S) started exhibiting this pathology only at about the 2-6 months after birth. In mice in which one of the endogenous allele was deleted (Casp-8-/+/BAC-C362S), the pathology became visible already at four days after birth, and developed rapidly. Histological analysis of skin samples (for details see Material and Methods) suggests inflammatory processes, which are manifested at 7 day postnatal but not 24 hours postnatal **(****Fig. 1D****).**

We found that expression of enzymatically deficient caspase-8, Casp-8BAC-C362S, interferes with the function of co-expressed proficient enzyme, and induces the development of a skin inflammatory disease, suggesting that the caspase-8 enzymatic function plays a role in the development or homeostasis of the skin after birth.

### Example 2: Caspase-8 conditional knock out mice confirm the role of the function of caspase-8 in skin disease development.

In order to validate that the skin epidermal pathology observed in the mice expressing the Casp8BAC-C362S trangene reflects a functional role of caspase-8 in the epidermal cells themselves, we employed mice with a conditional caspase-8 allele to delete caspase-8 specifically in these cells (for details see Materials and Methods). Crossing mice in which part of one of the caspase-8 alleles was flanked by loxP sites and the other allele was deleted (Casp-8^{fl/-}) with a transgenic mouse lane that expresses Cre under control of the kertatinocyte-specific Keratin-5 promoter resulted in effective and exclusive deletion of the caspase-8 gene in the epidermis as shown by PCR and Western blot analysis**(****Figs. 2A and 2B****).**

For Western blot analysis, skin was removed from P0 Casp-S^{fl/-}K5-Cre mice and incubated for 2-3 seconds at 65°C to separate epidermis and dermis. Both the epidermis and dermis samples were homogenized in RIPA buffer (50mM Tris-HCl, pH 8.0, 1% NP40, 0.5% sodium deoxycholate, 0.1% SDS, 150mM sodium chloride, 1mM EDTA, 1mM PMSF, 1mM sodium orthovanadate, complete protease inhibitor cocktail) and left on ice for 30min. Then cell homogenates were spun at 13000xg for 15min and supernatants were removed. 40 µg of protein extracts were resolved in 10% SDS-polyacryamide gel and blotted onto nitrocellulose membranes. The membrane was incubated with anti-mouse caspase-8 (3B10, 1:2000 dilution, Alexis) or anti-beta Actin (1:10000 dilution, Sigma) and detected with anti-Rat-HRP (Sigma) or anti-Mouse-HRP in a standard ECL reaction (Pierce) according to the manufacture's instruction. The results of the Western blot analysis summarized in **Fig. 2A** (lower panel) show that deletion of caspase 8 is manifested in the epidermis but not in the dermis of Casp-8^{fl/-}K5-Cre mice.

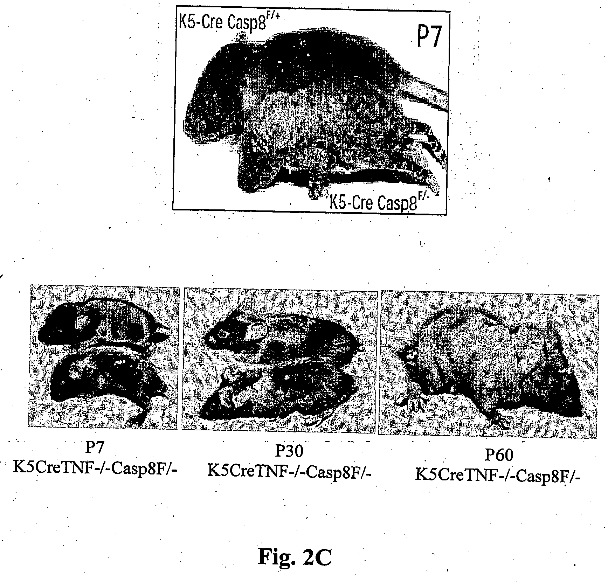

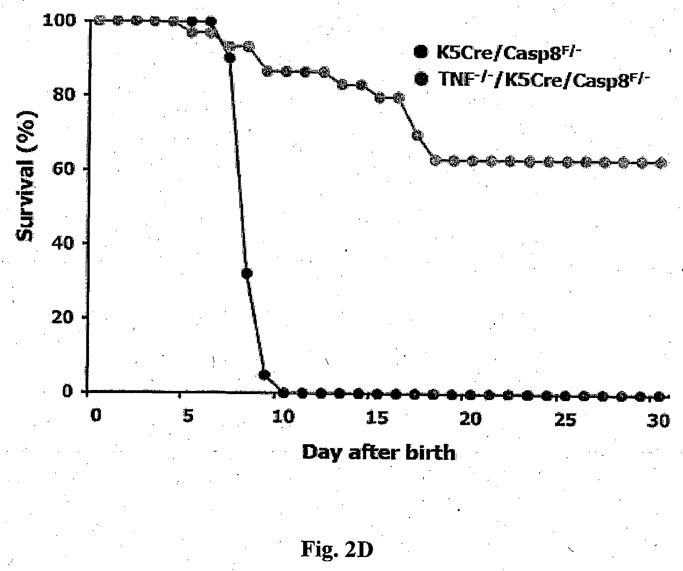

The mice (Casp-8^{fl/-}KS-Cre) appeared normal at birth. However, only 3 days later they started developing skin pathology similar to that of the Casp-8^{-/+}BAC-C362S mice **(****Fig. 2C****).** In spite of the homogenous expression of Cre and effective caspase-8 deletion in the epidermis, the lesions were initially focal, but then spread rapidly, eventually occupying the whole skin area. The Casp-8^{fl/-}K5-Cre mice were smaller then normal and survived only for a limited time **(****Fig. 2D****),** they all died at the 10th day of age. Consistent with the expression of the K5 promoter also in the epithelium of the stomach lining, part of the mice also displayed a severe pathology of the stomach and failed to eat.

In histological analysis (for details see Material and Methods), the epidermis of the Casp-8^{fl/-}K5-Cre mice were found to display, just as in the Casp-8^{-/+}/BAC-C362S mice, as well as features of inflammation such as accumulation of leukocytes in the dermis and expression of Keratin 6 in the epidermis (a marker for inflammatory and hyperproliferative condition) specifically at the site of the epithelial lesion. Furthermore, we found significant suprabasal expression of keratin 14 (K14) in the mutant skin, which is normally confined to the basal epidermal layer **(****Fig. 4A** and **Fig. 5****).** K6 and K14 were expressed in all viable layers of the knockout epidermis at P7 **(****Fig. 4A****).** In addition, at this stage, expression of the suprabasal keratin 1 (K1) and of the keratinocyte terminal differentiation markers loricrin and filaggrin was markedly reduced or altogether absent. The leukocytes accumulating in the dermis were mainly mononuclear phagocytes (F4/80 antibody staining, **Fig. 3B****)** and eosinophils (phenol-red positive, with no significance increase of either T or B lymphocytes (staining with anti-CD3 **(****Fig. 3B****),** CD45R or CD20 antibody, not shown). Some eosinophils also accumulated within pustules in the epidermis **(****Fig. 3B****).**

### Example 3: Contribution of TNF to the skin inflammatory disease associated with caspase-8 deficiency in keratinocytes.

Arrest of activation of the p65 NF-kB protein in the skin keratinocytes, by knockout of either p65 itself or of IKK2, the kinase mediating p65 NF-kB activation, also results in inflammatory skin disorder (Hu et al. 1999, Pasparakis et al. 2002). In that case, the disorder was shown to be triggered by chronic autocrine stimulation of the keratinocytes by TNF and a resulting enhancement of Jun phosphorylation, which, in the absence of a restraining effect of co-activated p65 occurs to an excessive extent. To explore the relationship between this skin inflammatory process and that which occurs as a result of caspase-8 deficiency in the keratinocytes, we assessed the contribution of TNF to the latter. Deletion of either the TNF gene or the TNF receptor 1 (TNFR1) gene in the Casp-8^{fl/-}K5-Cre mice by crossing them with the respective knockout mice resulted in a marked delay of initiation of the skin pathology **(****Fig. 2C****).** Moreover, the TNF-/-Casp-8^{fl/-}K5-Cre mice **(****Fig. 2D****)** and TNF R1-/-Casp-8^{fl/-}K5-Cre mice(not shown) remained viable for at least 4 months. A significant delay in initiation of the skin pathology could also be obtained by injecting anti-TNF antibodies or soluble TNF receptors to the Casp-8^{fl/-}K5-Cre mice (not shown). However, in contrast to the pathology resulting from deletion of p65 or IKK2 in the keratinocytes, which fully depends on TNF, the pathology resulting from caspase-8 deletion, although delayed, eventually also reached full-blown extent in the mice that did not express TNF or its receptor. In fact, since these mice did not die, the extent of skin pathology reached in them was far greater than that reached in the TNF proficient Casp-8^{fl/-}K5-Cre mice. We found by histological analysis that the skin lesion reached in the absence of TNF response was indistinguishable from that observed in its presence (not shown). Further deletion of the TNFR2 gene, besides that of TNFR1, had no effect on the skin pathology (not shown).

In further distinction from the effect of p65 inhibition, no increase in Jun phosphorylation could be observed in the epidermis of the Casp-8^{fl/-}K5-Cre mice **(****Fig. 4B****).** These findings suggested that caspase-8 deletion does not result in arrest of p65 NF-kB activation. Indeed, no difference could be discerned between the extents of p65 and p50 nuclear translocation in the epidermis of Casp-8^{fl/-}K5-Cre and normal mice (not shown).

Attempting to find out what role does caspase-8 serve in the skin keratinocytes, we first examined whether its deficiency affects cell death, a process with which caspase-8 is commonly associated. Tunnel tests revealed no decrease but rather significant increase of cell death in the caspase-8 deficient epidermis (**Fig. 4C****,** right panel). On assessment of cell-growth in the epidermis by staining for the Ki67 antigen we observed significant enhancement of cell growth in the epidermal basal layer (**Fig. 4C****,** left panel). However, in contrast to the overgrowth observed in NF-kB deficient epidermis, which involves significant thickening of the layer of dividing cells, the dividing cells in the caspase-8 deficient skin occupied just one, or at most two cell layers. Similar observations were reached when assessing cell growth in the skin by immunohistochemical assessment of bromo-deoxy uridine incorporation (not shown).

While the size of the epidermal basal layer remained unchanged, the pattern of expression of proteins that characterize distinct steps in differentiation of the keratinocytes was extensively altered in the caspase-8 deficient skin **(****Fig. 4A****).**

### Example 4: Ability of keratinocytes from the normal and caspase-8-deficient epidermis skin layers to differentiate in culture.

To find out if the aberrations of the cell differentiation pattern found in the caspase-8 deficient skin **(****Fig. 4A****)** reflect a cell autonomous function of caspase-8 or a more complex change involving interactions of the keratinocytes with other cells or with extracellular factors, we compared the ability of keratinocytes from the normal and caspase-8-deficient epidermis skin layers to differentiate in culture (for details see Material and Methods). Triggering differentiation of normal keratinocytes by increase of calcium ions results in characteristic morphologic changes as well as in effective induction of the early differentiation marker Keratin 1 and the late differentiation markers filgarin and loricrin. Calcium up-shift induced in the Casp-8^{fl/-}K5-Cre cells morphological changes similar to those observed in normal cells. However, it failed to induce in them increase in expression of keratin 1, filgarin or loricrin **(****Figs. 6****,** **7).** This differentiation deficiency was not observed with keratinocytes, obtained right after birth of the Casp-8^{fl/-}K5-Cre mice. However, this differentiation deficiency was evident with cells from the skin of one-day-old Casp-8^{fl/-}K5-Cre pups, in spite of the fact that at this age these pups did not manifest yet any abnormal skin histological feature. In spite of the extensive delay of the pathology in TNF-/-Casp-8^{fl/-}K5-Cre and TNF R1-/-Casp-8^{fl/-}K5-Cre mice, differentiation deficiency was observed in their cells too, already one day after birth.

p21ARF, a nuclear protein participating in cell growth and differentiation control was shown to decrease dramatically in differentiating keratinocytes (Dotto 2000 and Di Cunto et al. 1998). This change appears to have a causative role in the gene-expression changes associated with the differentiation. By determining the amounts of p21ARF in the cultured keratinocytes we found that its decrease following induction of differentiation by calcium up-shift is significantly delayed in the caspase-8 deficient cell, indicating further that this deficiency interferes with some aspects of the differentiation process **(****Fig. 7B****).**

Thus, while cultured in *vitro,* caspase-8 knockout keratinocytes grow normally and withdraw from the cell cycle following application of elevated calcium. However, the molecular events normally associated with late differentiation steps fail to occur in the knockout culture. Most notably, expression of loricrin and filaggrin is not induced. Furthermore, p21 is not degraded when cells enter late differentiation stage.

To further validate that the contribution caspase-8 to the expression of keratinocyte differentiation markers occurs in a cell-autonomous manner, caspase-8 is reconstituted in the Casp-8^{fl/-}K5-Cre kerartinocytes. In cultured Casp-8^{fl/-}K5-Cre kertinocytes the expression of wild type caspase-8 is reinstituted using a lenti-virus expression vector and the ability to express the differentiation markers in response to calcium up-shift is regained. No such response to calcium up-shift is observed when the cells are transfected with an enzymatically inactive caspase-8 mutant (Casp-8 -C362S). Moreover, expression of this mutant in keratinocytes derived from wild-type mice arrest the expression of the differentiation markers in them. Similar inhibition of differentiation marker expression is observed when keratinocytes derived from wild-type mice are transfected with a caspase-8 inhibitory protein or are treated with an agent capable of inhibiting caspase-8 activation. These results confirm that caspase-8 plays a cell-autonomous role in skin keratinocyte differentiation and indicates that this role depends on the enzymatic function of caspase-8.

### Example 5: Gene array analysis of skin from The Casp-8^{fl/-}K5-Cre mice and from their F/+ littermates three days after birth.

A mouse oligo microarray Gene kit (Catalog 60-mer Oligo) purchased from Agilent Technologies was used following the manufacturer's instructions with RNA samples from skin of the Casp-8^{fl/-}K5-Cre and from their F/+ littermates at three days after birth (P3). We found that the following genes with known pro-inflammatory functions were significantly upregulated in skin of Cre caspase-8fl/- mice: ISG15 (G1p2), Cxc110, 9230117N10Rik (IL33)-human orthologue is called C90rf26, IL-19, Sprr2f, S100a9, IL-6, MMP13, Ccl3, and IL1b. The differential pattern of expression in samples of skin from the Casp-8^{fl/-}K5-Cre mice shows predominant upregulation of type TH2 cytokines.

It was also found that two cDNAS of unknown function, 9230117E20Rik (SEQ ID NO: 2), and 2010002N04Rik (SEQ ID NO: 3) were significantly upregulated in the Casp-8^{fl/-}K5-Cre mice (Table 1).

**Table 1. Genes with unknown functions upregulated in the c8-null skin at P3 (fold increase in two independent microarray experiments is shown).**

| Accession | Exp. 1 | Exp. 2 | cDNA | Available Annotation |
|---|---|---|---|---|
| 9230117E20Rik | 12.4 | 7.09 | SEQ ID NO: 2 | Protease Inhibitor domain containing protein |
| 2010002N04Rik | 3.88 | 3.10 | SEQ ID NO: 3 | putative small membrane protein NID67 |

### Example 6: Differentially up-regulated genes in samples of skin of the Casp-8^{fl/-}K5-Cre mice assessed by real time PCR and in-situ hybridization.

The differential pattern of gene regulation found in the gene array analysis of the preceding Example was further evaluated by real time PCR. For this purpose, skin was removed from wild type mice, Casp-8^{fl/-}K5-Cre mice, or the double knockout TNF-/- Casp-8^{fl/-}K5-Cre mice at different times after birth. Caspase-8-defficient mice were tested as follows: a day before birth (D-1), at the day of birth (P0), one day after birth (P1), two days after birth (P2), three days after birth (P3), and five days after birth (P5). Skin removed from mice was incubated for 2-3 seconds at 65°C to separate epidermis and dermis. The sample of epidermis and dermis was converted into powder (with the aid of a mortar and pestle) and the powder was used as the starting material for the isolation of RNA with the Rneasy Kit of Quiagen, used following the manufacturer's instructions. RNA isolated from each skin sample removed from wild type mice, Casp-8^{fl/-}K5-Cre mice, or the double knockout TNF-/- Casp-8^{fl/-}K5-Cre mice at different times after birth was subjected to real time PCR using specific primers for genes which were found to be upregulated by the array technology.

All PCR reactions were carried out on Applied Biosystems 7500 Real-Time PCR System using proprietary TaqMan Gene Expression Assays obtained commercially and according to the manufacturer (Applied Biosystems) instructions.

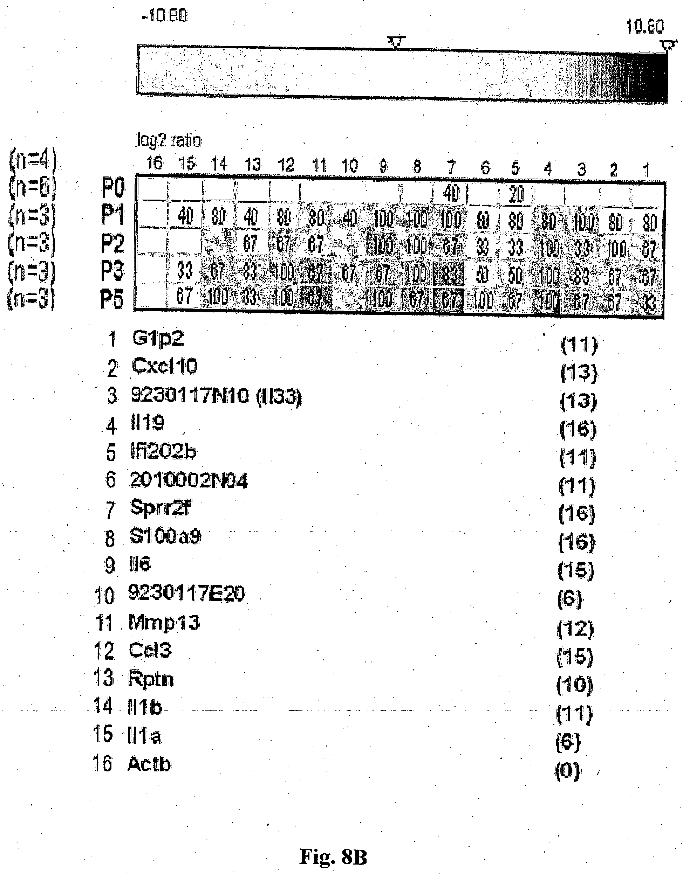

The mRNA level found for each specific gene by PCR was normalized with the mRNA level of the control gene beta-actin (ACTB) tested in the same PCR reaction. mRNA upregulation of a specific gene at a given developmental stage after birth in 8^{fl/-}K5-Cre mice or the double knockout TNF-/- Casp-8^{fl/-}K5-Cre mice was calculated as the ratio of normalized mRNA of 8^{fl/-}-K5-Cre mice or the double knockout TNF-/- Casp-8^{fl/-}K5-Cre mice and normalized mRNA of wild type found at the same developmental stage after birth by the delta Ct-method (Livak and Scmittgen, 2001). The ratios obtained were log2-transformed and ordered according to increasing consistency of up-regulation of expression changes as shown in the heatmap of **Fig. 8****,** in which each column shows the percentage of upregulation of a particular gene in the skin of 8^{fl/-}-K5-Cre mice before birth and at P0; P1, P2, P3 and P5. The results show that out of the 22 8^{fl/-}K5-Cre mice tested 19 showed significant upregulation of Isg15 (G1p2), 15 of CXC110, 15 of 9230117N10Rik (IL33), 14 of IL-19, 14 of 2010002N04Rik (SEQ ID NO: 3), 13 of Sprr2f, 13 of S100a9, 12 of IL-6, 12 of 9230117E20Rik (SEQ ID NO: 2), 11 of MM.P13, 11 of Ccl3, and 10 of IL1b gene.

The results show that out of the 22 double knockout TNF-/- Casp-8^{fl/-}K5-Cre mice tested 11 showed significant upregulation of Isg 15 (G1p2), 13 of CXC110, 13 of 9230117N10Rik (IL33), 16 of IL-19, 11 of 2010002N04Rik (SEQ ID NO: 3), 16 of Sprr2f, 16 of S100a9, 16 of IL-6, 6 of 9230117E20Rik (SEQ ID NO: 2), 12 of MMP13, 15 of Ccl3, and 11 of IL1b gene.

As mentioned, the skin pathology develops at day 3 after birth in the neck and the head area and gradually spreads to the whole body. We found by RT-PCR the same pattern of differential expression in samples from normal skin and skin showing pathology of the same P3 8^{fl/-}K5-Cre mice (not shown).

The results obtained by RT-PCR analysis confirmed that genes having pro-inflammatory functions and genes encoding type TH2 cytokines such as IL-19 are significantly upregulated in samples of skin from Casp-8^{fl/-}K5-Cre mice, that the same pattern of upregulation can be detected before the pathology develops, and that TNF has no principal effect on skin pathology, it only serves to amplify and expedite its development.

The effect of caspase-8 deficiency on the skin was further evaluated by in situ hybridization with a probe for s100a9 (see below), a chemokine found to be up regulated in the gene array analysis and in RT-PCR analysis of samples of skin from Casp-8^{fl/-}K5-Cre mice. For this purpose, paraffinized skin sections of 6 um thick from P7 wild type mice or P7 Casp-8^{fl/-}K5-Cre mice were applied in slides. After deparaffinization (by subjecting to xylene 10 minutes 2 times, 100% ethanol 2 times for 2minutes of each, 95% ethanol 2minutes, 70% ethanol 2minutes, 50% ethanol 2minutes), slides were treated with proteinase K (10ug/ml) for 10∼20min, fixed with 4% paraformaldehyde (PFA) and acetylated (employing 2.2ml triethanolamine in 200ml DEPC treated water with 750ul of acetic anhydride for 5 minutes). Then slides were then hybridized with anti-sense or sense (control) probe for overnight incubation at 65°C and 24 hours latter, the slides were washed with several different concentrations of SSC buffer [a. 5X SSC with 10mM DTT 30min at 65C, b. 2X SSC with 10mM DTT and 50% formamide 30min at 65C, c. 2X SSC 5min x 3 at 37C, d. RNase A (10ug/ml) in 0.4M NaCl, 0.01M Tris-HCl pH7.5, 0.005M EDTA 30min at 37C, and e. 2X SSC 15min at 37C, f. 0.1X SSC 15min at 37C] and incubated with anti-DIG antibody for 2hr at room temperature. After that, the slides were developed by a colorimetric substrate (Anti-Digoxigenin-Alkaline Phosphatase, Roche) for few hours to several days.

The results of in situ hybridization with anti-sense (SEQ ID NO: 35) and sense (control) probes from s100a9 applied to skin of P7 wild type mice or of P7 Casp-8^{fl/-} K5-Cre mice are summarized in **Fig. 9****.** The results obtained show that the s100a9 transcript is detected only in samples of Casp-8^{fl/-}K5-Cre mice probed with anti-sense probe in both the epidermis and dermis.

### Example 7: Differentially up-regulated proteins and activation of caspase-14, Stat-1 and Stat-3 in skin of Casp-8^{fl/-}K5-Cre mice assessed by Western blot hybridization.

For Western blot analysis, samples of whole skin, epidermis and dermis, of P4 Casp-8^{fl/-}K5-Cre mice or P4 wild type mice was isolated. Each sample was crushed with pestle and mortar and cells in the tissue were lysed by incubation with 10ul of RIPA buffer for 1mg of tissue (RIPA buffer, 20mM Tris-HCl pH 7.5, 150mM NaCl, 1mM EDTA pH8, 0.1 % SDS, 1% NP-40) was used for extraction of protein. Next, the sample of tissue lysate was incubated for 10min at 0°C, sample buffer added, and incubated at 100°C for 5min. Samples were span 13,00rpm and the supernatant resolved on 10% SDS polyacryamide gel and blotted onto nitrocellulose membranes.

The nitrocellulose membranes containing the resolved skin proteins were probed with antibodies specific for Stat-1 and Stat-3. Stat-1 and Stat-3 are transcription factors that became active upon phosphorylation, by protein kinases that are induced by IL-6, translocate to the nucleus and are used as markers for skin inflammatory pathology. The results obtained with the Stat specific antibodies show that only in the samples of skin epidermis of Casp-8^{fl/-}K5-Cre mice a band corresponding to phosphorylated Stat-1 or phosphorylated Stat-3 is detected **(****Fig. 10A****).**

Since pro-Caspase-14 is known to be constitutively processed into active caspase-14 in the skin epidermis, it was of interest to test whether the resulting inflammatory pathology induced by caspase-8 deficiency involves regulation of pro-caspase-14 processing in this tissue. For this purpose, the nitrocellulose membranes containing the resolved skin proteins were probed with antibodies specific for caspase-14. The results summarized on **Fig**. **10B** shows that the processing of pro-caspase-14 in the skin of Casp-8^{fl/-}K5-Cre mice was not altered indicating that caspase-14 is not involved in the pathogenesis induced by caspase-8 deficiency.

S100A8 is a chemokine expressed by macrophages. S100A8 is known to form an heterodimer with S100A9, a gene that we previously found to be upregulated in the skin of Casp-8^{fl/-}K5-Cre mice by gene array and in the epidermis of Casp-8^{fl/-}K5-Cre mice by RT-PCR analysis. The presence of S100A8 protein was analyzed by Western blot hybridization in samples of whole skin, dermis or epidermis isolated from Casp-8^{fl/-}K5-Cre or wild type mice. The results summarized in **Fig. 10C** show that the S100A8 protein is detected only in Casp-8^{fl/-}K5-Cre mice and that the highest expression of S100A8 was found in the dermis. Thus, these results indicate that lack of caspase-8 in the epidermis triggers pathological changes in the dermis.

### Example 8: IL-1 a and IL-1 β do not contribute to the skin inflammatory disease associated with caspase-8 deficiency in keratinocytes.

IL-1 a and IL-1β were found to be upregulated in the skin of Casp-8^{fl/-}K5-Cre mice by gene array and in the epidermis of Casp-8^{fl/-}K5=Cre mice by RT-PCR analysis. We further explored the role of IL-1 a and IL-1 β in the skin inflammatory process, which occurs as a result of caspase-8 deficiency in the keratinocytes. Double knock out mice in either the IL-1 α gene or the IL-1 β gene and in the Casp-8^{fl/-}K5-Cre were obtained by crossing Casp-8^{fl/-}K5-Cre mice with the knock out mice in either the IL-1 α gene or the IL-1 β gene (Horai et al. 1998). The double knock out mice did not show a drastic delay of initiation of the skin pathology and survived only to day 8 or 9 as the Casp-8^{fl/-}K5-Cre mice.

### REFERENCES

Adams, J. C. and F. M. Watt. Changes in keratinocyte adhesion during terminal differentiation: reduction in fibronectin binding precedes alpha 5 beta 1 integrin loss from the cell surface. Cell 63(2): 425-35 (1990).
Bennett, N. T. and G. S. Schultz. Growth factors and wound healing: biochemical properties of growth factors and their receptors. Am J Surg 165(6): 728-37 (1993).
Candi E, Schmidt R, Melino G. The cornified envelope: a model of cell death in the skin.Nat Rev Mol Cell Biol. Apr;6(4):328-40. Review(2005).
Chaudhary PM, Eby MT, Jasmin A, Kumar A, Liu L, Hood L. Activation of the NF-kappaB pathway by caspase 8 and its homologs. Oncogene. 19:4451-60(2000).
Di Cunto, F., G. Topley, E. Calautti, J. Hsiao, L. Ong, P. K. Seth and G. P. Dotto. Inhibitory function of p21Cip1/WAF1 in differentiation of primary mouse keratinocytes independent of cell cycle control. Science 280(5366): 1069-72 (1998).
Dorsett, Y. and T. Tuschl. "siRNAs: applications in functional genomics and potential as therapeutics." Nat Rev Drug Discov 3(4): 318-29 (2004).
Dotto, G. P. p21(WAF1/Cip1): more than a break to the cell cycle? Biochim Biophys Acta 1471(1): M43-56 (2000).
Fuchs, E. and J. A. Segre Stem cells: a new lease on life. Cell 100(1): 143-55 (2000).
Gong S, Yang XW, Li C, Heintz N. "Highly efficient modification of bacterial artificial chromosomes (BACs) using novel shuttle vectors containing the R6Kgamma origin of replication" Genome Res. Dec; 12(12): 1992-8 (2002).
Hasegawa M, Imamura R, Kinoshita T, Matsumoto N, Masumoto J, Inohara N, Suda T. ASC-mediated NF-kappaB activation leading to interleukin-8 production requires caspase-8 and is inhibited by CLARP. J Biol Chem. 280:15122-30) (2005).
Horai R, Asano M, Sudo K, Kanuka H, Suzuki M, Nishihara M, Takahashi M, Iwakura Y. "Production of mice deficient in genes for interleukin (IL)-1alpha, IL-1beta, IL-1alpha/beta, and IL-1 receptor antagonist shows that IL-1beta is crucial in turpentine-induced fever development and glucocorticoid secretion" J Exp Med. May 4;187(9):1463-75 (1998).
Kang, T. B., T. Ben-Moshe, E. E. Varfolomeev, Y. Pewzner-Jung, N. Yogev, A. Jurewicz, A. Waisman, O. Brenner, R. Haffner, E. Gustafsson, P. Ramakrishnan, T. Lapidot and D. Wallach. Caspase-8 serves both apoptotic and nonapoptotic roles. J Immunol 173(5): 2976-84 (2004).
Kim, D. H., M. A. Behlke, et al. "Synthetic dsRNA Dicer substrates enhance RNAi potency and efficacy." Nat Biotechnol 23(2): 222-6 (2005).
Kupper TS, Fuhlbrigge RC.Immune surveillance in the skin: mechanisms and clinical consequences. Nat Rev Immunol. Mar;4(3):211-22. Review.(2004).
Lavker, R. M. and T. T. Sun Epidermal stem cells: properties, markers, and location. Proc Natl Acad Sci U S A 97(25): 13473-5 (2000).
Lippens, S., M. Kockx, M. Knaapen, L. Mortier, R. Polakowska, A. Verheyen, M. Garmyn, A. Zwijsen, P. Formstecher, D. Huylebroeck, P. Vandenabeele and W. Declercq Epidermal differentiation does not involve the pro-apoptotic executioner caspases, but is associated with caspase-14 induction and processing. Cell Death Differ. 7(12): 1218-24(2000).
Lippens, S., C. VandenBroecke, E. Van Damme, E. Tschachler, P. Vandenabeele and W. Declercq. Caspase-14 is expressed in the epidermis, the choroid plexus, the retinal pigment epithelium and thymic Hassall's bodies. Cell Death Differ 10(2): 257-9 (2003).
Livak,K.J. and Schmittgen.T.W. Analysis of relative gene expression data using real-time quantitative PCR and the 2-Ct method. Methods, 25, 402-408(2001).
Martin, P. Wound healing--aiming for perfect skin regeneration. Science 276(5309): 75-81(1997).
Okuyama, R., B. C. Nguyen, C. Talora, E. Ogawa, A. Tommasi di Vignano, M. Lioumi, G. Chiorino, H. Tagami, M. Woo and G. P. Dotto. High commitment of embryonic keratinocytes to terminal differentiation through a Notchl-caspase 3 regulatory mechanism. Dev Cell 6(4): 551-62 (2004).
Olivera-Martinez, I., J. P. Viallet, F. Michon, D. J. Pearton and D. Dhouailly. The different steps of skin formation in vertebrates. Int J Dev Biol 48(2-3): 107-15 (2004).
Pasparakis M, Courtois G, Hafner M, Schmidt-Supprian M, Nenci A, Toksoy A, Krampert M, Goebeler M, Gillitzer R, Israel A, Krieg T, Rajewsky K, Haase I. TNF-mediated inflammatory skin disease in mice with epidermis-specific deletion of IKK2.Nature. Jun 20;417(6891):861-6 (2002).
Peus, D. and M. R. Pittelkow. Growth factors in hair organ development and the hair growth cycle. Dermatol Clin 14(4): 559-72(1996).
Potten, C. S. Cell replacement in epidermis (keratopoiesis) via discrete units of proliferation. Int Rev Cytol 69: 271-318 (1981).
Ramirez, A., A. Bravo, J. L. Jorcano and M. Vidal. Sequences 5' of the bovine keratin 5 gene direct tissue-and cell-type-specific expression of a lacZ gene in the adult and during development. Differentiation 58(1): 53-64(1994).
Sohn, D., K. Schulze-Osthoff and R. U. Janicke. Caspase-8 can be activated by interchain proteolysis without receptor-triggered dimerization during drug-induced apoptosis. J Biol Chem 280(7): 5267-73(2005).
Soutschek, J., A. Akinc, et al. "Therapeutic silencing of an endogenous gene by systemic administration of modified siRNAs." Nature 432(7014): 173-8(2004).
Sparwasser T, Gong S, Li JY, Eberl G. "General method for the modification of different BAC types and the rapid generation of BAC transgenic mice" Genesis Jan;38(1):39-50(2004).
Takahashi, T., M. Ogo and T. Hibino. Partial purification and characterization of two distinct types of caspases from human epidermis. J Invest Dermatol 111(3): 367-72(1998).
Takeda K, Takeuchi O, Tsujimura T, Itami S, Adachi O, Kawai T, Sanjo H, Yoshikawa K, Terada N, Akira S. Limb and skin abnormalities in mice lacking IKKalpha. Science. Apr 9;284(5412):313-6(1999).
Varfolomeev, E. E., M. Schuchmann, V. Luria, N. Chiannilkulchai, J. S. Beckmann, I. L. Mett, D. Rebrikov, V. M. Brodianski, O. C. Kemper, O. Kollet, T. Lapidot, D. Soffer, T. Sobe, K. B. Avraham, T. Goncharov, H. Holtmann, P. Lonai and D. Wallach. Targeted disruption of the mouse Caspase 8 gene ablates cell death induction by the TNF receptors, Fas/Apo1, and DR3 and is lethal prenatally. Immunity 9(2): 267-76(1998).
Wallach, D., M. Boldin, E. Varfolomeev, R. Beyaert, P. Vandenabeele and W. Fiers. Cell death induction by receptors of the TNF family: towards a molecular understanding. FEBS Lett 410(1): 96-106 (1997).
Zhang JY, Green CL, Tao S, Khavari PA. NF-kappaB RelA opposes epidermal proliferation driven by TNFR1 and JNK. Genes Dev. Jan 1;18(1):17-22. Erratum in: Genes Dev. 2004 Feb 15;18(4):461(2004).

## Claims

1. Use of an agent selected from caspase-8 or a fragment of caspase-8 that retains biological activity of caspase-8, in the manufacture of a medicament for the treatment or prevention of an inflammatory skin disease, disorder or condition.

2. An agent selected from caspase-8 or a fragment of caspase-8 that retains the biological activity of caspase-8, for use in the treatment or prevention of an inflammatory skin disease, disorder or condition.

3. The use according to claim 1 or the agent for use according to claim 2, wherein the inflammatory skin disease is atopic dermatitis or psoriasis.

4. A method for diagnosing in an individual a skin disease, disorder or condition associated with caspase-8 deficiency in the skin or the predisposition to develop said skin disease, disorder or condition, comprising, measuring in a sample of skin of the tested individual and in a sample of skin of at least one healthy control individual the expression level or activity of caspase-8 and/or detecting aberrations in nucleic acid encoding caspase-8 in a sample of skin from the tested individual, wherein detection of a decrease of caspase-8 activity or expression level in the sample of the tested individual as compared to the sample of said at least one healthy control individual, and/or detection of aberrations in nucleic acid encoding caspase-8 in the tested individual is indicative of said skin disease, disorder or condition, or of a predisposition to develop said skin disease, disorder or condition in the tested individual.

5. The method according to claim 4, for diagnosing in an individual a skin disease, disorder or condition associated with caspase-8 deficiency in epithelial keratinocytes or the predisposition to develop said skin disease, disorder or condition.

6. A method for generating a non-human animal model of an inflammatory skin disease, disorder or condition, comprising developing a non-human animal arrested in keratinocyte caspase-8 expression level or activity.

7. The method according to claim 6, wherein the arrest of caspase-8 expression level is effected by knocking out caspase-8 in epithelial keratinocyte of the non-human animal.

8. The method according to claim 6, wherein the arrest of caspase-8 activity is effected by inducing transgenic expression of caspase-8 mutant lacking enzymatic activity in the non-human animal.

9. The method according to claim 8, wherein the transgenic caspase-8 mutant is a bacterial artificial chromosome with mutant caspase-8 lacking enzymatic activity, in which the Cys at residue 362 has been replaced for Ser.

10. The method according to any one of claims 6 to 9, wherein the non-human animal is mouse.

11. A method for identifying a target protein involved in the pathology or course of a skin disease, disorder or condition whose level of expression or activity is normally regulated by caspase-8 activity in the skin comprising the steps of: comparing the profile of gene expression in a sample of skin comprising epithelial keratinocytes arrested in caspase-8 expression level or activity to the profile of gene expression in a sample of skin comprising epithelial keratinocytes having normal caspase-8 expression level or activity, wherein a gene whose expression is downregulated or upregulated in the sample comprising epithelial keratinocytes arrested in caspase-8 expression level or activity encodes a candidate target protein involved in the pathology or course of said skin disease, disorder or condition.

12. The method according to claim 11, wherein the skin disease, disorder or condition is an inflammatory skin disease, disorder or condition.

13. The method according to any one of claims 4 to 9 or 12, wherein the disease is atopic dermatitis or psoriasis.

14. A method of screening of a candidate compound for treating or preventing a skin inflammatory disease, disorder or condition comprising, providing a culture of cells comprising keratinocytes arrested in caspase-8 expression level or activity, introducing a test compound in said cells, inducing differentiation of the cells by increasing the calcium concentration in the culture medium of the cells, measuring the expression level of a differentiation marker of the skin and/or p21ARF in the presence or absence of the test compound, wherein increase in the expression level of a differentiation marker of the skin and/or decrease of p21ARF in the presence of the test compound is indicative that the test compound is a candidate compound for treating or preventing said disease, disorder or condition.

15. A method according to claim 14, wherein the differentiation marker is selected from keratin 1, filagrin, and loricrin.

16. A method for testing the efficacy of a candidate compound for treating a skin inflammatory disease, disorder or condition, comprising administering to a non-human animal model generated according to any one of claims 6 to 10 the candidate test compound and assessing prevention or reduction of skin pathology in said animal model.

## Patentansprüche

1. Verwendung eines Mittels, das aus Caspase-8 oder einem Fragment von Caspase-8 ausgewählt ist, das eine biologische Aktivität von Caspase-8 beibehält, bei der Herstellung eines Medikaments zur Behandlung oder Prävention einer entzündlichen Hautkrankheit, einer entzündlichen Hautstörung oder eines entzündlichen Hautzustands.

2. Mittel, das aus Caspase-8 oder einem Fragment von Caspase-8 ausgewählt ist, das die biologische Aktivität von Caspase-8 beibehält, zur Verwendung bei der Behandlung oder Prävention einer entzündlichen Hautkrankheit, einer entzündlichen Hautstörung oder eines entzündlichen Hautzustands.

3. Verwendung nach Anspruch 1 oder Mittel zur Verwendung nach Anspruch 2, wobei die entzündliche Hautkrankheit atopische Dermatitis oder Psoriasis ist.

4. Verfahren zum Diagnostizieren einer Hautkrankheit, einer Hautstörung oder eines Hautzustands, die bzw. der mit einem Caspase-8-Mangel in der Haut assoziiert ist, oder der Veranlagung für die Entwicklung der Hautkrankheit, der Hautstörung oder des Hautzustands bei einem Individuum, das das Messen der Expressionshöhe oder Aktivität von Caspase-8 in einer Hautprobe des getesteten Individuums und in einer Hautprobe zumindest eines gesunden Kontrollindividuums und/oder das Nachweisen von Abweichungen von Nukleinsäure, die für Caspase-8 codiert, in einer Hautprobe des getesteten Individuums, umfasst, wobei der Nachweis einer Verringerung der Caspase-8-Aktivität oder -Expressionshöhe in der Probe des getesteten Individuums wie mit der Probe des zumindest einen gesunden Kontrollindividuums verglichen, und/oder der Nachweis von Abweichungen von Nukleinsäure, die für Caspase-8 codiert, beim getesteten Individuum für die Hautkrankheit, die Hautstörung oder den Hautzustand und/oder für eine Veranlagung zur Entwicklung der Hautkrankheit, der Hautstörung oder des Hautzustands beim getesteten Individuum indikativ ist.

5. Verfahren nach Anspruch 4 zum Diagnostizieren einer Hautkrankheit, einer Hautstörung oder eines Hautzustands, die bzw. der mit einem Caspase-8-Mangel in Epithelkeratinozyten assoziiert ist, oder der Veranlagung für die Entwicklung der Hauptkrankheit, der Hautstörung oder des Hautzustands bei einem Individuum.

6. Verfahren zum Erzeugen eines nicht-humanen Tiermodells zu einer entzündlichen Hautkrankheit, einer entzündlichen Hautstörung oder eines entzündlichen Hautzustands, das das Entwickeln eines nicht-humanen Tiers umfasst, dessen Keratinozyten-Caspase-8-Expressionshöhe oder -Aktivität eingefroren ist.

7. Verfahren nach Anspruch 6, wobei das Einfrieren der Caspase-8-Expressionshöhe durch ein Knockout von Caspase-8 in Epithelkeratinozyten des nicht-humanen Tiers bewirkt wird.

8. Verfahren nach Anspruch 6, wobei das Einfrieren der Caspase-8-Aktivität durch Induzieren einer transgenen Expression einer Caspase-8-Mutante, der es an enzymatischer Aktivität mangelt, bei dem nicht-humanen Tier bewirkt wird.

9. Verfahren nach Anspruch 8, wobei die transgene Caspase-8-Mutante ein künstliches bakterielles Chromosom mit einer mutierten Caspase-8, der es an enzymatischer Aktivität fehlt, ist, wobei das Cys an Rest 362 durch Ser ersetzt wurde.

10. Verfahren nach einem der Ansprüche 6 bis 9, wobei das nicht-humane Tier eine Maus ist.

11. Verfahren zum Identifizieren eines Zielproteins, das an der Pathologie oder dem Verlauf einer Hautkrankheit, einer Hautstörung oder eines Hautzustands beteiligt ist, dessen Expressionshöhe oder Aktivität für gewöhnlich durch die Caspase-8-Aktivität in der Haut reguliert wird, das die Schritte umfasst: Vergleichen des Genexpressionsprofils in einer Hautprobe, die Epithelkeratinozyten mit eingefrorener Caspase-8-Expressionshöhe oder -Aktivität umfasst, mit dem Genexpressionsprofil in einer Hautprobe, die Epithelkeratinozyten mit normaler Caspase-8-Expressionshöhe oder -Aktivität umfasst, wobei ein Gen, dessen Expression in der Probe, die Epithelkeratinozyten mit eingefrorener Caspase-8-Expressionshöhe oder -Aktivität umfasst, runter- oder hochreguliert ist, für ein Kandidaten-Zielprotein codiert, das an der Pathologie oder dem Verlauf der Hautkrankheit, der Hautstörung oder des Hautzustands beteiligt ist.

12. Verfahren nach Anspruch 11, wobei die Hautkrankheit, die Hautstörung oder der Hautzustand eine entzündliche Hautkrankheit, eine entzündliche Hautstörung oder ein entzündlicher Hautzustand ist.

13. Verfahren nach einem der Ansprüche 4 bis 9 oder 12, wobei die Krankheit atopische Dermatitis oder Psoriasis ist.

14. Verfahren zum Screenen einer Kandidatenverbindung zur Behandlung oder Prävention einer entzündlichen Hautkrankheit, einer entzündlichen Hautstörung oder eines entzündlichen Hautzustands, das das Bereitstellen einer Kultur von Zellen, die Keratinozyten mit eingefrorener Caspase-8-Expressionshöhe oder -Aktivität umfassen, das Einbringen einer Testverbindung in die Zellen, das Induzieren einer Differenzierung der Zellen durch Erhöhen der Calciumkonzentration im Kulturmedium der Zellen, das Messen der Expressionshöhe eines Differenzierungsmarkers der Haut und/oder von p21ARF in Gegenwart oder Abwesenheit der Testverbindung umfasst, wobei eine Erhöhung der Expressionshöhe eines Differenzierungsmarkers der Haut und/oder eine Abnahme von p21ARF in Gegenwart der Testverbindung dafür indikativ ist, dass die Testverbindung eine Kandidatenverbindung zur Behandlung oder Prävention der Krankheit, der Störung oder des Zustands ist.

15. Verfahren nach Anspruch 14, wobei der Differenzierungsmarker aus Keratin 1, Filagrin und Loricrin ausgewählt ist.

16. Verfahren zum Testen der Wirksamkeit einer Kandidatenverbindung zur Behandlung einer entzündlichen Hautkrankheit, einer entzündlichen Hautstörung oder eines entzündlichen Hautzustands, das das Verabreichen der Kandidaten-Testverbindung an ein nicht-humanes Tiermodell, das nach einem der Ansprüche 6 bis 10 erzeugt wurde, und das Bewerten der Prävention oder Verringerung von Hautpathologie im Tiermodell umfasst.

## Revendications

1. Utilisation d'un agent sélectionné parmi la caspase-8 ou un fragment de la caspase-8 qui conserve l'activité biologique de la caspase-8, dans la fabrication d'un médicament destiné au traitement ou à la prévention d'une maladie, d'un trouble ou d'une affection cutané(e) inflammatoire.

2. Agent sélectionné parmi la caspase-8 ou un fragment de la caspase-8 qui conserve l'activité biologique de la caspase-8, destiné à être utilisé dans le traitement ou la prévention d'une maladie, d'un trouble ou d'une affection cutané(e) inflammatoire .

3. Utilisation selon la revendication 1 ou agent destiné à être utilisé selon la revendication 2, où la maladie cutanée inflammatoire est la dermatite atopique ou le psoriasis.

4. Procédé pour diagnostiquer chez un individu une maladie, un trouble ou une affection cutané(e) associé(e) à un déficit en caspase-8 dans la peau ou la prédisposition à développer ladite maladie, ledit trouble ou ladite affection cutané(e), comprenant la mesure, dans un échantillon de peau de l'individu testé et dans un échantillon de peau d'au moins un individu de contrôle sain, du taux d'expression ou de l'activité de la caspase-8 et/ou la détection d'aberrations dans un acide nucléique codant pour la caspase-8 dans un échantillon de peau de l'individu testé, où la détection d'une réduction de l'activité ou du taux d'expression de la caspase-8 dans l'échantillon de l'individu testé par comparaison à l'échantillon dudit au moins un individu de contrôle sain, et/ou la détection d'aberrations dans un acide nucléique codant pour la caspase-8 chez l'individu testé, indique ladite maladie, ledit trouble ou ladite affection cutané(e), ou une prédisposition à développer ladite maladie, ledit trouble ou ladite affection cutané(e) chez l'individu testé.

5. Procédé selon la revendication 4, pour diagnostiquer chez un individu une maladie, un trouble ou une affection cutané(e) associé(e) à un déficit en caspase-8 dans les kératinocytes de l'épithélium ou la prédisposition à développer ladite maladie, ledit trouble ou ladite affection cutané(e).

6. Procédé pour générer un modèle animal non humain d'une maladie, d'un trouble ou d'une affection cutané(e) inflammatoire, comprenant le développement d'un animal non humain chez qui le taux d'expression ou l'activité de la caspase-8 des kératinocytes est arrêté(e).

7. Procédé selon la revendication 6, dans lequel l'arrêt du taux d'expression de la caspase-8 est obtenu par l'inactivation de la caspase-8 dans les kératinocytes de l'épithélium de l'animal non humain.

8. Procédé selon la revendication 6, dans lequel l'arrêt de l'activité de la caspase-8 est obtenu par l'induction de l'expression transgénique d'un mutant de la caspase-8 dépourvu d'activité enzymatique chez l'animal non humain.

9. Procédé selon la revendication 8, dans lequel le mutant de la caspase-8 transgénique est un chromosome artificiel bactérien comportant une caspase-8 mutante dépourvue d'activité enzymatique, dans laquelle la Cys au résidu 362 a été remplacée par Ser.

10. Procédé selon l'une quelconque des revendications 6 à 9, dans lequel l'animal non humain est une souris.

11. Procédé pour identifier une protéine cible impliquée dans la pathologie ou l'évolution d'une maladie, d'un trouble ou d'une affection cutané(e) dont le taux d'expression ou l'activité est normalement régulé(e) par l'activité de la caspase-8 dans la peau, comprenant les étapes consistant à : comparer le profil d'expression génique dans un échantillon de peau comprenant des kératinocytes de l'épithélium dans lesquels le taux d'expression ou l'activité de la caspase-8 est arrêté(e) avec le profil d'expression génique dans un échantillon de peau comprenant des kératinocytes de l'épithélium présentant un taux d'expression ou une activité de la caspase-8 normal(e), où un gène dont l'expression est négativement régulée ou positivement régulée dans l'échantillon comprenant des kératinocytes de l'épithélium dans lesquels le taux d'expression ou l'activité de la caspase-8 est arrêté(e) code pour une protéine cible candidate impliquée dans la pathologie ou l'évolution de ladite maladie, dudit trouble ou de ladite affection cutané(e).

12. Procédé selon la revendication 11, dans lequel la maladie, le trouble ou l'affection cutané(e) est une maladie, un trouble ou une affection cutané(e) inflammatoire.

13. Procédé selon l'une quelconque des revendications 4 à 9 ou 12, dans lequel la maladie est la dermatite atopique ou le psoriasis.

14. Procédé de criblage d'un composé candidat pour le traitement ou la prévention d'une maladie, d'un trouble ou d'une affection cutané(e) inflammatoire, comprenant la mise à disposition d'une culture de cellules comprenant des kératinocytes dans lesquels le taux d'expression ou l'activité de la caspase-8 est arrêté(e), l'introduction d'un composé de test dans lesdites cellules, l'induction de la différenciation des cellules en augmentant la concentration du calcium dans le milieu de culture des cellules, la mesure du taux d'expression d'un marqueur de différenciation de la peau et/ou de p21ARF en présence ou en l'absence du composé de test, où une augmentation du taux d'expression d'un marqueur de différenciation de la peau et/ou une réduction de p21ARF en présence du composé de test indique que le composé de test est un composé candidat pour le traitement ou la prévention de ladite maladie, dudit trouble ou de ladite affection.

15. Procédé selon la revendication 14, dans lequel le marqueur de différenciation est sélectionné parmi la kératine 1, la filaggrine et la loricrine.

16. Procédé pour tester l'efficacité d'un composé candidat à traiter une maladie, un trouble ou une affection cutané(e) inflammatoire, comprenant l'administration, à un modèle animal non humain généré selon l'une quelconque des revendications 6 à 10, du composé de test candidat et l'évaluation de la prévention ou de la réduction de la pathologie cutanée chez ledit modèle animal.


### SEQUENCE LISTING

<110> Yeda Research and Development Co. Ltd
   WALLACH, David
   KIM, Jin chul
   KANG, Tae Bong
   KOVALENKO, Andrei
<120> PHARMACEUTICAL COMPOSITIONS AND DIAGNOSTIC METHODS FOR INFLAMMATORY SKIN DISEASES, DISORDERS OR CONDITIONS
<130> 1002
<140> 171451
   <141> 2005-10-16
<150> 171451
   <151> 2005-10-16
<160> 35
<170> PatentIn version 3.1
<210> 1
   <211> 2526
   <212> DNA
   <213> Mus musculus
<400> 1
   gagaaatcac ggcagaatca tcgagaaacc tgaaaaatga gacctagaat gaagtattcc     60
   aactccaaga tttccccggc aaagttcagc agcaccgcag gcgaagccct ggtcccgcct    120
   tgcaaaataa gaagatccca acagaagacc aaagaattct gccatgtcta ctgcatgaga    180
   ctccgttctg gcctcaccat aagaaaggag actagttatt ttaggaaaga acccacgaaa    240
   agatattcac taaaatcggg taccaagcat gaagagaact tctctgccta tccacgggat    300
   tctaggaaga gatccttgct tggcagtatc caagcatttg ctgcgtctgt tgacacattg    360
   agcatccaag gaacttcact tttaacacag tctcctgcct ccctgagtac atacaatgac    420
   caatctgtta gttttgtttt ggagaatgga tgttatgtga tcaatgttga cgactctgga    480
   aaagaccaag agcaagacca ggtgctacta cgctactatg agtctccctg tcctgcaagt    540
   caatcaggcg acggtgtgga tgggaagaag gtgatggtga acatgagtcc catcaaagac    600
   acagacatct ggctgcatgc caacgacaag gactactccg tggagcttca aaggggtgac    660
   gtctcgcctc cggaacaggc cttcttcgtc cttcacaaaa agtcctcgga ctttgtttca    720
   tttgaatgca agaatcttcc tggcacttac ataggagtaa aagataacca gctggctcta    780
   gtggaggaga aagatgagag ctgcaacaat attatgttta agctctcgaa aatctaatgc    840
   agtaaaacgc ctgtgcgttc tgggttgaat gacttaatgc ttccaactga agaaagggta    900
   acagagagaa agaaagccat tcttggctta cgatgttgtg gaatgttatt atgtagaaaa    960
   cttcttttat ttccttttct tcagctacat gttcaataga ctcacagata tatgacttac   1020
   ggcgttggta aagaaactga aggagattca gccttgctct ttccttttct ctgccttgag   1080
   tcctgtatga aatcacactc acggacttca gaagagcagg caccacagtg catggtttgc   1140
   tttagagagg gtccatttca aaaaccttca taaaaacaat gcaaaacaag aaaacaaccg   1200
   aacaaaaaaa ccacctattt cctggttcta aacaaatgat tgtaatacta gagcagttag   1260
   tgggaggacc agctaggggg aggatcacct aggggaggac cagctagggg gaggaccagc   1320
   tgctgcaaag actgactgtt tctcacttat aataaaatgc caaatgcctc cgcagatgcc   1380
   ccaggcaacc ctcagatcag ccctttctgt gaagagtggc gttacctgtg cttgtttcct   1440
   tcttaaactt ccaatttttc tcttttaaca catttaacat ttaactttaa gcaagccagc   1500
   ttacattagg aagtgaaaga cattttagtt cccacccgtg attgaaatca ttgactatat   1560
   ctaacaagct taaagtctcc tgtaagaact gatcaggata tacactagga catgccaata   1620
   gaatgggatc tcatggtgca gtctgaagcc ctccaactgg agagacgcta acatcatcct   1680
   tctcgctgat ttccaaggag ctatgacttt ggatgcatgc atctgcttgg atgagatgtc   1740
   tcggctgctt gctttcctta tgcacacgtt ctgttcagct tcacagcagc aatgctcacg   1800
   gtggaatagc ttagcttagc ttctgcccct tctttggttc ttttgaccac catatccgta   1860
   acggctctcc tactccctca gctttctttc tctttgctct gacgtctata tgccaacaca   1920
   cttattccac tgtctttacc ctgcactgca gaattttaca tctacctact ggttaccagg   1980
   ttgtcccctg aacaaccttc ctttgtgttt tactgttatt aaagtagtaa tatttgtatt   2040
   caaccatgta gtaatatttt aagccactaa aggaatagtt ttacttattt agacaacagc   2100
   aatttctact acatttttat aagcttaaaa cttacatgtt ttaaaactta aaacgataaa   2160
   gacaataaca acattgatgg agtatgatat gacagttcag aaagggttag ctcttatctt   2220
   ccagtcgagg aaacctattg tatacaatag ctggaggaat gtatgatcaa agaggccggg   2280
   aaccgccgtg taggatcgta cggctgtaac aggtataatt gtttcattaa tttgtcacag   2340
   tcttactgta gaggaatgta aaggcggaat ctgcgtcatt cctctggaaa ccacagtgtt   2400
   gactctgtga atctgtacga tatctttaaa gtagtaacta cgtagtcaaa tgtgttcttg 2460
   acgttgttca taactttgaa taaaccattt ttcaaaacca cgtgtgacca caaaaaaaaa 2520
   aaaaaa 2526
<210> 2
   <211> 497
   <212> DNA
   <213> Mus musculus
<400> 2
   gacttataaa tacagtccag agaacaccat cctgcatgtt caacgcccct aacatttctg 60
   tgttctcaga cctcaagatt ctcagcgcca tgaagccagc aggtgccttt ctgcttttga 120
   tcagcctggc ctgtttgttc ctctctgtag atgctgtgag ccaaggaggc tttcaggctt 180
   tttgcagcaa ctatgagaag acactggccc cagatggaaa atcatgccca aagacccaca 240
   aacctgtgtg tggcactgat ggaaaaacct accaaaaccg ctgtgcattc tgccaaacag 300
   ccatggaaag aagtttgggg aaacttggtt tcaaacatga agggaaatgc tgattctcga 360
   acatcacaat actgggtgtg cctttgcttt gctttttaca ctaagacatg tattccttat 420
   tacccagttt ccacagcagt gtgcttccca ccaattatta cgcataaaac aaaataaata 480
   aagctatgcc agtcact 497
<210> 3
   <211> 1550
   <212> DNA
   <213> Mus musculus
<400> 3
   agcacgttct cggcgctagc ggggctgagg ttccagagct ggggttgctt gaggatccct 60
   ttcgatccta ggggcccaga aaagcaccgg ttactgcttg taatcctgac tgaagatcgg 120
   gatcggatct aggggccaac atggacgcta tcacccaatc ccctgtggat gccgtgcttc 180
   ccaagcacat cctggatatc tgggccatag tcctcatcat cctggctacc attgtcatca 240
   tgacctcctt gtttctgtgc ccggccactg cggtcatcat ctatcgaatg cggactcacc 300
   cagttctcaa cggggctgcc tgagagagac ccttgaaggc gggatggcgt gtgataaggg 360
   cagaggtctt gcccctatcc tgatttcaga aagacagcgg ggagactcag gacctggatt 420
   tgtgctttaa ttttgattct gctgttgggc tacagtgtag atgtcatcaa gggacctaac 480
   tctgagtccc aggctctcct tagctttcag ccgtgggtca cgacccctgc cttcctacct 540
   cacagggttg tgggaaccac gtgacagcgg aggtaaaagc tgtttgagag cagtgaagcc 600
   acacagacct tgtgcagtga gaagctttga agaatcaaag acccctgaag ctgctgccgg    660
   tacaggaggc gagggcggct gggaaagagg aactttggcc agtgcccctc atgccgagcc    720
   aagcaaaatg gtgcacccca gccccagcaa atcagagttt tgtttttgtt tttgttttca    780
   agtgacactc tttccatttt tcttagtttg ggagaaagag cgagtatggc aggcgagaga    840
   gatgagtaaa agtttcagaa gcgtctcagc caatccctgc tcctgaagga gagctttgcc    900
   cttatgcacc aggcaagatg gcctgctttc agcactgggc cgcacatctc tgttagcgct    960
   gactctcagc cgggttagaa atgcaggacg gctgagtttt ctgccccgca tttcagctcc   1020
   tttatgtgcc ctacagtaga ggatttcccc tgtctgagct ttgtggcatt tagcttcaag   1080
   cttttgttat tactcttttg cggcaccaga gatggaactt tggcctcctt tacgctggga   1140
   aaggatccca ccactgagct acaacccagc ccttctagcc cccacagctg tctgaagtac   1200
   ttattatttt aagagatttc ccaggacttt tgaacccgac ttaagacaac gacggttatt   1260
   ttagatgcta tgatttatat ttatatagag atatttctag actattaaag ctcgcccttt   1320
   gatactggaa tcaggggcat cttgggattt attgttatgt aagaagatag catagatttt   1380
   gggatagtat tatgtcagac gttgcttact ctgatcttca ttgatgtatg catcccgttt   1440
   ccaataaatg ccacactgag agtgtctgaa gcttttcttt gaagaacacc tactgaactt   1500
   tgaacttaga taaaaagatt caactcaaaa aaaaaaaaaa aaaaaaaaaa              1550
<210> 4
   <211> 266
   <212> PRT
   <213> Mus musculus
<400> 4
   Met Arg Pro Arg Met Lys Tyr Ser Asn Ser Lys Ile Ser Pro Ala Lys
   1               5                   10                  15
   Phe Ser Ser Thr Ala Gly Glu Ala Leu Val Pro Pro Cys Lys Ile Arg
               20                  25                  30
   Arg Ser Gln Gln Lys Thr Lys Glu Phe Cys His Val Tyr Cys Met Arg
           35                  40                  45
   Leu Arg Ser Gly Leu Thr Ile Arg Lys Glu Thr Ser Tyr Phe Arg Lys
       50                  55                  60
   Glu Pro Thr Lys Arg Tyr Ser Leu Lys Ser Gly Thr Lys His Glu Glu
   65                  70                  75                  80
   Asn Phe Ser Ala Tyr Pro Arg Asp Ser Arg Lys Arg Ser Leu Leu Gly
                   85                  90                  95
   Ser Ile Gln Ala Phe Ala Ala Ser Val Asp Thr Leu Ser Ile Gln Gly
   100 105 110
   Thr Ser Leu Leu Thr Gln Ser Pro Ala Ser Leu Ser Thr Tyr Asn Asp
   115 120 125
   Gln Ser Val Ser Phe Val Leu Glu Asn Gly Cys Tyr Val Ile Asn Val
   130 135 140
   Asp Asp Ser Gly Lys Asp Gln Glu Gln Asp Gln Val Leu Leu Arg Tyr
   145 150 155 160
   Tyr Glu Ser Pro Cys Pro Ala Ser Gln Ser Gly Asp Gly Val Asp Gly
   165 170 175
   Lys Lys Val Met Val Asn Met Ser Pro Ile Lys Asp Thr Asp Ile Trp
   180 185 190
   Leu His Ala Asn Asp Lys Asp Tyr Ser Val Glu Leu Gln Arg Gly Asp
   195 200 205
   Val Ser Pro Pro Glu Gln Ala Phe Phe Val Leu His Lys Lys Ser Ser
   210 215 220
   Asp Phe Val Ser Phe Glu Cys Lys Asn Leu Pro Gly Thr Tyr Ile Gly
   225 230 235 240
   Val Lys Asp Asn Gln Leu Ala Leu Val Glu Glu Lys Asp Glu Ser Cys
   245 250 255
   Asn Asn Ile Met Phe Lys Leu Ser Lys Ile
   260 265
<210> 5
   <211> 105
   <212> PRT
   <213> Mus musculus
<400> 5
   Met Phe Asn Ala Pro Asn Ile Ser Val Phe Ser Asp Leu Lys Ile Leu
   1 5 10 15
   Ser Ala Met Lys Pro Ala Gly Ala Phe Leu Leu Leu Ile Ser Leu Ala
   20 25 30
   Cys Leu Phe Leu Ser Val Asp Ala Val Ser Gln Gly Gly Phe Gln Ala
   35 40 45
   Phe Cys Ser Asn Tyr Glu Lys Thr Leu Ala Pro Asp Gly Lys Ser Cys
   50 55 60
   Pro Lys Thr His Lys Pro Val Cys Gly Thr Asp Gly Lys Thr Tyr Gln
   65 70 75 80
   Asn Arg Cys Ala Phe Cys Gln Thr Ala Met Glu Arg Ser Leu Gly Lys
   85 90 95
   Leu Gly Phe Lys His Glu Gly Lys Cys
   100 105
<210> 6
   <211> 60
   <212> PRT
   <213> Mus musculus
<400> 6
   Met Asp Ala Ile Thr Gln Ser Pro Val Asp Ala Val Leu Pro Lys His
   1 5 10 15
   Ile Leu Asp Ile Trp Ala Ile Val Leu Ile Ile Leu Ala Thr Ile Val
   20 25 30
   Ile Met Thr Ser Leu Phe Leu Cys Pro Ala Thr Ala Val Ile Ile Tyr
   35 40 45
   Arg Met Arg Thr His Pro Val Leu Asn Gly Ala Ala
   50 55 60
<210> 7
   <211> 21
   <212> DNA
   <213> Artificial sequence
<220>
   <223> Primers
<400> 7
   ttagcatcct gactggcgtg a 21
<210> 8
   <211> 23
   <212> DNA
   <213> Artificial sequence
<220>
   <223> Primers
<400> 8
   aagccatgtg aactgtggag agc 23
<210> 9
   <211> 20
   <212> DNA
   <213> Artificial sequence
<220>
   <223> Primers
<400> 9
   gaagacctgg ctgccctcaa 20
<210> 10
   <211> 20
   <212> DNA
   <213> Artificial sequence
<220>
   <223> Primers
<400> 10
   ggatcccgca gctctctcac 20
<210> 11
   <211> 20
   <212> DNA
   <213> Artificial sequence
<220>
   <223> Primers
<400> 11
   ctctagggct ggcaccagga 20
<210> 12
   <211> 20
   <212> DNA
   <213> Artificial sequence
<220>
   <223> Primers
<400> 12
   ccggctcaca gaggtttgct 20
<210> 13
   <211> 33
   <212> DNA
   <213> Artificial sequence
<220>
   <223> Primers
<400> 13
   ggcgcgccta catacgccta ggaaggaccc tgt 33
<210> 14
   <211> 60
   <212> DNA
   <213> Artificial sequence
<220>
   <223> Primers
<400> 14
   gaattcgacg gcggacgagg aggtgtctgc ctatgactct gttgcttgcc tttggattcc 60
<210> 15
   <211> 60
   <212> DNA
   <213> Artificial sequence
<220>
   <223> Primers
<400> 15
   cctcgtccgc cgtcgaattc ccagttattg gagaacccac atgaagacca agctgcacat 60
<210> 16
   <211> 43
   <212> DNA
   <213> Artificial sequence
<220>
   <223> Primer
<400> 16
   aaggaaaaaa gcggccgcgt tactcaggtt acgtggggga aag 43
<210> 17
   <211> 45
   <212> DNA
   <213> Artificial sequence
<400> 17
   gcacgcgtcg acggcgcgcc ggtcttgatt cagtgacttt caact 45
<210> 18
   <211> 34
   <212> DNA
   <213> Artificial sequence
<220>
   <223> Primer
<400> 18
   ggaattctta gggagggaag aagagcttct tccg 34
<210> 19
   <211> 31
   <212> DNA.
   <213> Artificial sequence
<220>
   <223> Primer
<400> 19
   ggaattcggg agggaagaag agcttcttcc g 31
<210> 20
   <211> 31
   <212> DNA
   <213> Artificial sequence
<220>
   <223> Primer
<400> 20
   gactagttga tgtgtgctct ccacagttca c 31
<210> 21
   <211> 43
   <212> DNA
   <213> Artificial sequence
<220>
   <223> Primer
<400> 21
   aaggaaaaaa gcggccgccc cattctgtta accagattca tgc 43
<210> 22
   <211> 20
   <212> DNA
   <213> Artificial sequence
<220>
   <223> Primer
<400> 22
   cctcgtccgc cgtcgaattc 20
<210> 23
   <211> 20
   <212> DNA
   <213> Artificial sequence
<220>
   <223> primer
<400> 23
   ccctcacctc tgtgcctgct 20
<210> 24
   <211> 20
   <212> DNA
   <213> Artificial sequence
<220>
   <223> Primer
<400> 24
   gctcggggag tcttgtggaa 20
<210> 25
   <211> 29
   <212> DNA
   <213> Artificial sequence
<220>
   <223> Primer
<400> 25
   aggcgcgcct gcctacaggc tgtgttctg 29
<210> 26
   <211> 48
   <212> DNA
   <213> Artificial sequence
<220>
   <223> Primer
<400> 26
   aaggaaaaaa gcggccgcct ctaaccacag aaatgagtaa ggaagcat 48
<210> 27
   <211> 22
   <212> DNA
   <213> Artificial sequence
<220>
   <223> Primer
<400> 27
   tggtttcttg taaccagcag ag 22
<210> 28
   <211> 20
   <212> DNA
   <213> Artificial sequence
<220>
   <223> Primer
<400> 28
   agacccctag gaatgctcgt 20
<210> 29
   <211> 20
   <212> DNA
   <213> Artificial sequence
<220>
   <223> Primer
<400> 29
   acatggtcct gctggagttc 20
<210> 30
   <211> 20
   <212> DNA
   <213> Artificial sequence
<220>
   <223> Primer
<400> 30
   attcacccca ttctgctgac 20
<210> 31
   <211> 35
   <212> DNA
   <213> Artificial sequence
<220>
   <223> Primer
<400> 31
   ttggcgcgcc tctatgcaag agcagcaagg actct 35
<210> 32
   <211> 35
   <212> DNA
   <213> Artificial sequence
<220>
   <223> Primer
<400> 32
   ttgcggccgc gctgttctgg caactcagtt ttctc 35
<210> 33
   <211> 36
   <212> DNA
   <213> Artificial sequence
<220>
   <223> Primers
<400> 33
   cctttctgga agttacttcc ttgggaagct tgaatg 36
<210> 34
   <211> 36
   <212> DNA
   <213> Artificial sequence
<220>
   <223> Primer
<400> 34
   cattcaagct tcccaaggaa gtaacttcca gaaagg 36
<210> 35
   <211> 300
   <212> DNA
   <213> Artificial- Probe
<400> 35
   gccaacaaag caccttctca gatggagcgc agcataacca ccatcatcga caccttccat    60
   caatactcta ggaaggaagg acaccctgac accctgagca agaaggaatt cagacaaatg   120
   gtggaagcac agttggcaac ctttatgaag aaagagaaga gaaatgaagc cctcataaat   180
   gacatcatgg aggacctgga cacaaaccag gacaatcagc tgagctttga ggagtgtatg   240
   atgctgatgg caaagttgat ctttgcctgt catgagaagc tgcatgagaa caacccacgt   300